# EUROPEAN PATENT SPECIFICATION

(11) **EP 2 361 634 B1**
(45) Date of publication and mention of the grant of the patent: **21.10.2015**
(21) Application number: 10290094.1
(22) Date of filing: 25.02.2010
(51) Int. Cl.: A61K 39/00, A61K 39/395, C07K 14/435

(54) **Proteins/peptides having immunogenic properties and their biological applications**
Proteine/Peptide mit immunogenen Eigenschaften und deren biologische Anwendungen
Protéines/peptides dotés de propriétés immunogènes et leurs applications biologiques

(43) Date of publication of application: 31.08.2011
(73) Proprietor: Institut de Recherche pour le Développement, 13572 Marseille Cedex 02 (FR)
(72) Inventor: Misse Brumas, Marthe Dorothee, 34820 Teyran (FR)
(74) Representative: Cabinet Armengaud Aîné

(56) References cited:
- VALENZUELA J G ET AL: "Toward a description of the sialome of the adult female mosquito Aedes aegypti." September 2002 (2002-09), INSECT BIOCHEMISTRY AND MOLECULAR BIOLOGY SEP 2002 LNKD- PUBMED:12213246, VOL. 32, NR. 9, PAGE(S) 1101 - 1122 , XP002594406 ISSN: 0965-1748 * abstract *
- ARCA ET AL: "An insight into the sialome of the adult female mosquito Aedes albopictus" INSECT BIOCHEMISTRY AND MOLECULAR BIOLOGY, ELSEVIER SCIENCE LTD, GB LNKD- DOI:10.1016/J.IBMB.2006.10.007, vol. 37, no. 2, 20 January 2007 (2007-01-20), pages 107-127, XP005855349 ISSN: 0965-1748
- COCIANCICH S O ET AL: "Vesicular ATPase-overexpressing cells determine the distribution of malaria parasite oocysts on the midguts of mosquitoes." 30 April 1999 (1999-04-30), THE JOURNAL OF BIOLOGICAL CHEMISTRY 30 APR 1999 LNKD- PUBMED:10212245, VOL. 274, NR. 18, PAGE(S) 12650 - 12655 , XP002594407 ISSN: 0021-9258 * abstract; figure 2 *
- JAMES A A ET AL: "Isolation and characterization of the gene expressing the major salivary gland protein of the female mosquito, Aedes aegypti" MOLECULAR AND BIOCHEMICAL PARASITOLOGY, ELSEVIER SCIENCE PUBLISHERS, AMSTERDAM, NL LNKD- DOI:10.1016/0166-6851(91)90010-4, vol. 44, no. 2, 1 February 1991 (1991-02-01), pages 245-253, XP023896959 ISSN: 0166-6851 [retrieved on 1991-02-01]
- HUANG CHIN-GI ET AL: "Intestinal expression of H+ V-ATPase in the mosquito Aedes albopictus is tightly associated with gregarine infection." March 2006 (2006-03), THE JOURNAL OF EUKARYOTIC MICROBIOLOGY 2006 MAR-APR LNKD- PUBMED:16579815, VOL. 53, NR. 2, PAGE(S) 127 - 135 , XP002594408 ISSN: 1066-5234 * page 130 - column 2 *
- NENE VISHVANATH ET AL: "Genome sequence of Aedes aegypti, a major arbovirus vector." 22 June 2007 (2007-06-22), SCIENCE (NEW YORK, N.Y.) 22 JUN 2007 LNKD- PUBMED:17510324, VOL. 316, NR. 5832, PAGE(S) 1718 - 1723 , XP002594409 ISSN: 1095-9203 * the whole document *
- RIBEIRO JOSÉ MC ET AL: "An annotated catalogue of salivary gland transcripts in the adult female mosquito, AEdes aegypti*" BMC GENOMICS, BIOMED CENTRAL, LONDON, GB LNKD- DOI:10.1186/1471-2164-8-6, vol. 8, no. 1, 4 January 2007 (2007-01-04) , page 6, XP021022366 ISSN: 1471-2164
- PENG ZHIKANG ET AL: "Immunoblot analysis of salivary allergens in 10 mosquito species with worldwide distribution and the human IgE responses to these allergens", April 1998 (1998-04), JOURNAL OF ALLERGY AND CLINICAL IMMUNOLOGY, VOL. 10, NR. 4 PART 1, PAGE(S) 498-505 ISSN: 0091-6749
- SIMONS F E ET AL: "Mosquito allergy: recombinant mosquito salivary antigens for new diagnostic tests.", January 2001 (2001-01), INTERNATIONAL ARCHIVES OF ALLERGY AND IMMUNOLOGY 2001 JAN-MAR LNKD- PUBMED:11307029, VOL. 124, NR. 1-3, PAGE(S) 403 - 405 ISSN: 1018-2438
- REMOUE F ET AL: "Evaluation of the antibody response to Anopheles salivary antigens as a potential marker of risk of malaria", TRANSACTIONS OF THE ROYAL SOCIETY OF TROPICAL MEDICINE AND HYGIENE, ELSEVIER, GB, vol. 100, no. 4, 1 April 2006 (2006-04-01) , pages 363-370, XP027985205, ISSN: 0035-9203 [retrieved on 2006-04-01]

## Description

The invention relates to proteins/peptides having immunogenic properties, such as obtained from *Aedes* mosquitoes, particularly from salivary gland extracts of *Ae.aegypti* or *Ae.albopictus.*

Dengue virus (DV) causes dengue fever and the more severe conditions dengue hemorrhagic fever (DHF) and dengue shock syndrome (DSS). The World Health Organization estimates that there are 50 million dengue infections every year worldwide. *Aedes aegypti* and *Aedes albopictus,* common vectors of DV, are both found in Thailand together in some areas where they pose a major risk of epidemic DHF.

An understanding of the mosquito's normal physiology could help with controlling its pathogenic state. Mosquitoes feed on sugar to sustain life, and the females also feed on blood to obtain the nutrients necessary to produce yolk proteins and eggs; during such blood meals, pathogens can be transmitted from the insect vector to a vertebrate host.

Pathogens such as DV pass through the insect's midgut (MG) epithelial cells into the lymph and thence to other organs. They ultimately reach the salivary gland (SG) where virus propagation takes place, with subsequent transfer to a vertebrate host at the next feeding.

The MG, a site of nutrient digestion and absorption, is also an important immune site, but the molecular mechanisms that prevent pathogen infection in mosquito MG is less understood. It has been proposed that the infection barriers in the mosquitoes are the midgut infection barrier, which prevents DV invasion of MG epithelial cells, and the MG escape barrier, which prevents the dissemination of DV to other tissues.

Valenzuela et al. (Insect Biochemistry and Molecular Biology, 2002, vol. 32, 1101-1122) describe the sequencing of 456 *Ae.aegypti* cDNA sequences belonging to 238 sequence clusters and informatics research for the role of the cDNA sequences.

Arca et al. (Insect Biochemistry and Molecular Biology, 2007, vol. 37, 107-127) describe the sequencing of 1021 Ae.albopictus cDNA sequences belonging to 446 sequence clusters.

Cociancich et al. (The journal of biology chemistry, 1999, vol. 274, p12650-12655) describe that anti-V-ATPase subunit B serum is produced in a rabbit by injecting recombinant V-ATPase subunit B.

Huang et al. (The journal of eukaryotic microbiology, 2006, vol. 53, p127-135) describe the production of polyclonal antibody directed to V-ATPase in a rabbit with the help of recombinant V-ATPase subunit A. Said antibody is used to detect V-ATPase in the midgut and Malpighian tubules of mosquito larvae.

The work of James et al. (Molecular and biochemical parasitology, 1991, vol. 44, p245-253) concerns the study relating to protein D7 of Ae. aegypti. Polyclonal anti-recombinant D7 antibody was produced in a rabbit by injecting recombinant D7 protein.

Nene et al. (Science, 2007, vol. 316, p1718-1723) report an analysis of Aedes aegypti genome. However, this document does not give any detail teaching about immunogenic properties of mosquito salivary gland proteins.

The article of Ribeiro et al. (BMC Genomics, 2007, vol. 8, p6) is related to the analysis of 614 transcripts expressed in adult female Ae.aegypti mosquitoes, in order to identify putative secreted proteins expressed in the salivary gland of Ae.aegypti. The functions or putative functions of these proteins are discussed in this document

Peng et al. (Journal of allergy and clinical immunology, 1998, vol.10, 498-505) is focused on the study of relationship between mosquito salivary proteins and human allergy to mosquito bites.

Simons et al. (International archives of allergy and immunology, 2001, vol. 124, 403-405) is also for the objective to provide recombinant mosquito salivary antigens giving skin test positivity for the diagnosis of mosquito allergy.

Currently, only the proteomic analysis of larvae *Ae. aegypti* midgut brush border membrane vesicles is available. Knowing that only adult females can transmit pathogens, the MG protein profile of the adult females must be further characterized in order to understand the physiological function of this organ.

The mosquito SG is the last organ in the vector with which viruses are in contact before being inoculated into a vertebrate host, and SG-derived factors may enhance virus transmission. The SG plays an important role in food digestion as well as transmission of pathogens. Mosquito saliva is vital for successful blood feeding because it contains anticoagulant, anti-inflammatory, and immunosuppressive factors. Saliva proteins are also antigenic and immunogenic, involving immunoglobulin E, immunoglobulin G, and T-lymphocyte-mediated hypersensitivity response.

In an approach for studying the physiological processes that appear during a blood meal, the inventors have developped means to identify the physiological differences between sugar-fed (SF) and blood-fed (BF) mosquitoes. Using proteomics, they have, first, elaborated an *Ae. aegypti* protein map of MG and SG by using 2D-PAGE coupled to MS, and second, compared blood-fed (BF) and sugar-fed (SF) mosquitoes to identify candidate proteins potentially involved in pathogen invasion of mosquitoes and transmission to vertebrate hosts. Thus, 2-D DIGE coupled with mass spectrometry (MS) was employed to analyze and compare SG and MG proteomes of *Ae. aegypti* adult females to identify proteins with altered levels within these two organs between BF and SF female mosquitoes. It was thus observed that certain SG proteins were highly expressed only in BF mosquitoes. In SF mosquitoes, housekeeping proteins were highly expressed (especially those related to energy metabolism) and actin was up-regulated. For the first time, the 2-DE profiles of immunogenic *Ae. aegypti* SG BF-related proteins were generated giving new data useful for the understanding of the physiological processes that appear during the blood meal and for developing new tools useful for therapeutical applications..

These proteins/peptides having immunogenic properties, such as obtained from *Aedes* mosquitoes, particularly from salivary gland extracts of *Ae.aegypti* or *Ae.albopictus,* are analysed by using a method comprising
- submitting said extracts to a two-dimensional electrophoresis, staining the gels with a protein staining agent, followed by a Western blot, wherein the gels have been labelled or coloured to specifically detect the proteins/peptides,
- detecting IgG proteins on membranes with a pool of DHF patients' sera.

These proteins/peptides are proteins/peptides of sequences SEQ ID N° 1-9, 12-14, 16,18,21-24.
Proteins/peptides of sequences SEQ ID N°1-12 are particularly obtained from *Ae.aegypti.*
   They respectively correspond to
   SEQ ID N°1: *-Heat shock cognate 70 (Q1HQZ5_AEDAE)*
   SEQ ID N°2: *ATP synthase subunit beta vacuole (Q9XYC8)*
   SEQ ID N°3: *Putative adenosine deaminase (Q8T9T6_AEDAE)*
   SEQ ID N°4: *Salivary apyrase precurssor (P50635 APY_AEDAE)*
   SEQ ID N°5: *Salivary apyrase (Q176L8_AEDAE)*
   SEQ ID N°6: *Putative serpin (Q8T9U7*_*AEDAE)*
   SEQ ID N°7: *Salivary anti-FXa serpin (QIHRU9_AEDAE)*
   SEQ ID N°8: *Fibrinogen and fibronectin (Q17NC0_AEDAE)*
   SEQ ID N°9: *Angiopoietin-like protein variant (Q1HRV2_AEDAE)*
   SEQ ID N° 12: *Long form D7Bclul salivary protein d7l1 (Q5MIW7_AEDAL)*
   SEQ ID N° 13: *putative uncharacterized protein (62 kDa,Q17NC2_AEDAE)*
   SEQ ID N° 14: *putative uncharacterized protein (Q17F11_AEDAE)*
Proteins/peptides of sequences SEQ ID N°16, 18 and 21-24 are particularly obtained from *Ae. albopictus.*
   They respectively correspond to
   SEQ ID N°16: *D7 salivary Protein d7S6 (Q5MIP1_AEDAL)*
   SEQ ID N° 18: *V-type proton ATPase catalytic subunit A (O16109*/*VATA_AEDAL)*
   SEQ ID N°21: *Salivary antigen-5 related protein AG5-4 (Q5MIT3)*
   SEQ ID N° 22: *Putative salivary serpin (Q5MIW4)*
   SEQ ID N°23: *Putative adenosine deaminase (Q5MIX2_AEDAL)*
   SEQ ID N°24: *Gelsolin (Q177L3_AEDAE)*

SEQ ID N°25 and 26. SEQ ID N°25 is the *Aedes aegypti* equivalent of QSMIT3 identified in *Aedes albopictus.* SEQ ID N°26 has 99% of homology with Q17NCE.

The proteins/peptides having the above sequences have been identified by mass spectrometry, particularly by Maldi-TOF SM analysis. They can be obtainable from salivary glands of mosquitoes or by genetic engenering recombination, for example in a baculovirus system.

The proteins/peptides of sequences 13-15, 17 and 19-20, respectively correspond to:
SEQ ID N°10: *Putative 34 kDa family secreted salivary (Q1HRW0_AEDAE)*
SEQ ID N°11: *D7 protein precursor (ALL2_AEDAE)*
SEQ ID N° 15: *putative 30 kDa allergen like protein (Q8T9T8_AEDAE)*
SEQ ID N° 17: *sal ivary secredted protein34k-2 (Q5MIU2_AEDAL)*
SEQ ID N° 19: *salivary apyrase (QSMIX3_AEDAL)*
SEQ ID N° 20: *putative salivary secreted protein62 k-2 (Q5MIV0_AEDAL)*
The object of the invention is the use of at least one of peptide chosen from the peptide of sequence SEQ ID NO 1-15 as *in vitro* epidemiological indicator of exposure to dengue. Advantageously, the object of the invention concerns the use of the peptide of sequence SEQ ID NO:1 as *in vitro* epidemiological indicator of exposure to dengue.

It is disclosed herein that pharmaceutical compositions can comprise a therapeutically effective amount of at least one of the above defined proteins/peptides of sequences SEQ ID N°1-26, more particularly of sequences SEQ ID 1-9, 12-14, 16, 18, 21-26, in association with a pharmaceutically inert carrier.

Said pharmaceutical compositions are under appropriate forms for an administration by the oral, injectable or topic route.

For oral administration, the above defined pharmaceutical compositions are presented, for example, in the form of tablets, pills, capsules, drops, patchs or sprays.
For an administration by injection, the pharmaceutical compositions are under the form of solutions for injections by the intravenous, subcutaneous, or intramuscular route.

These pharmaceutical compositions may also be under the form of gels for a topic administration.

The above described compositions are particularly useful for preventing or treating patients susceptible to be exposed to *Aedes* mosquitoes bits or patients with viral infections such as dengue infections.

The doses will be chosen by the one skilled in the art taking into account the age and condition of the patient and the severity of the infection.

It is also disclosed herein the use of the proteins/peptides of sequences SEQ ID N° 10, 11, 15, 17 and 19-20 for making immune response immunomodulators for treating or preventing viral infections.

A method for treating a viral infection, such as dengue infection, by administering to a patient in need thereof therapeutically effective amount of at least one of the above defined proteins/peptides in association with a pharmaceutically inert carrier is also disclosed.

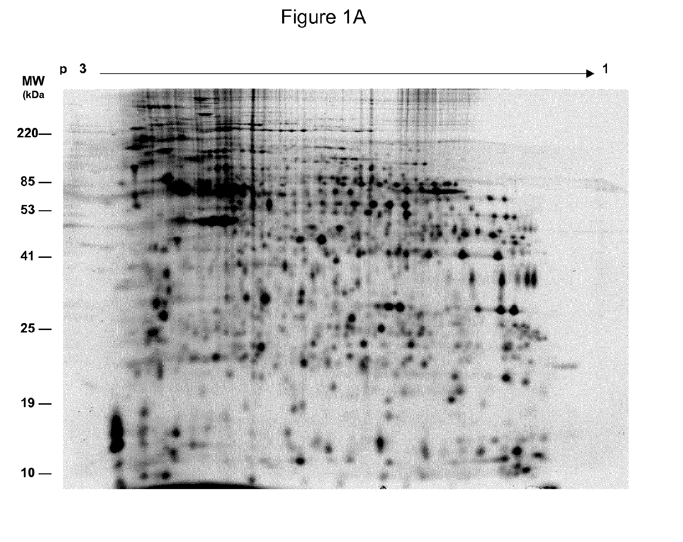

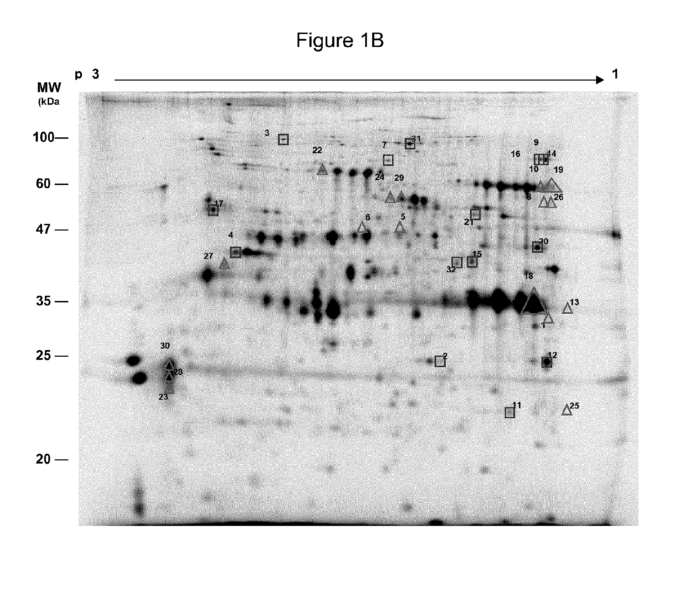

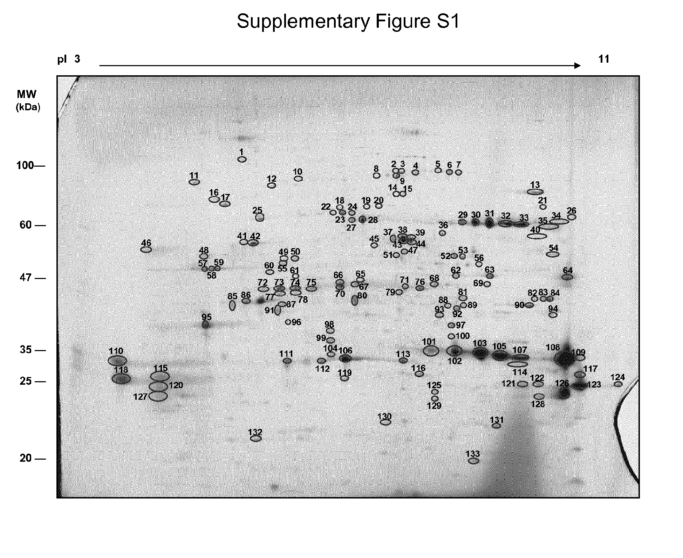

Other characteristics and advantages of the invention are given in the following Examples with reference to Figures 1-4 and S1 which represent, respectively:
- **Figure 1**. 2D-DIGE synthetic gel of *Ae. aegypti* MG (day 3) (A) and SG (day 10) (B) proteins for the SF and BF groups with a pI scale of 3-10. The molecular weight scales are indicated in the Figures. Protein spots differentially expressed in MG and SG are indicated by number and symbols: SF (□), BF (Δ).

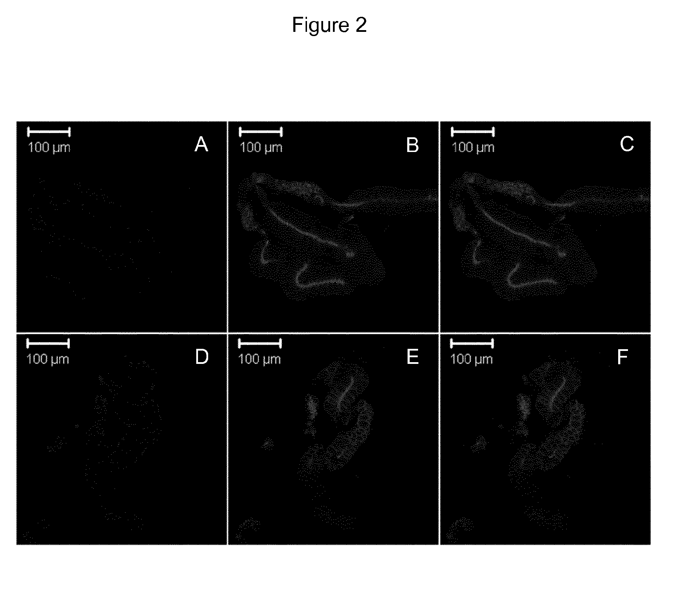

- **Figure 2****.** Expression of actin *i*n *Ae. aegypti* SG for SF and BF groups. Actin was detected in red by IFA at 10 days post sugar meal (A-C) or blood meal (D-F). Nuclei were marked with Hoechst in blue. Similar results were observed in three separate experiments.

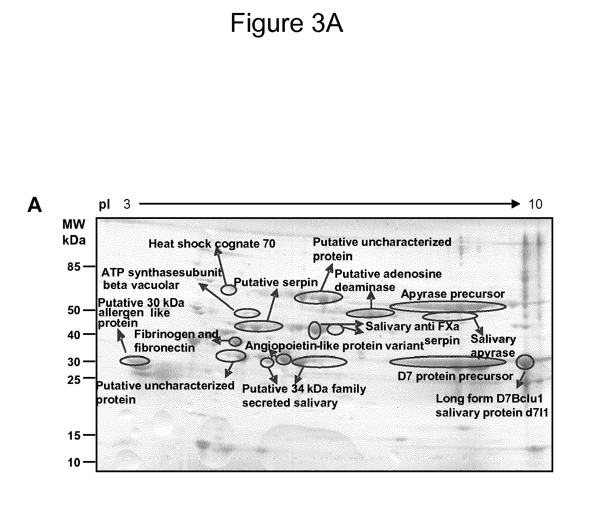

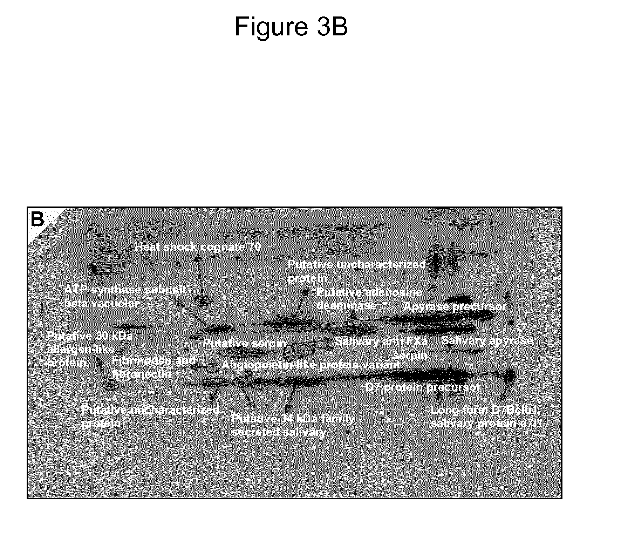

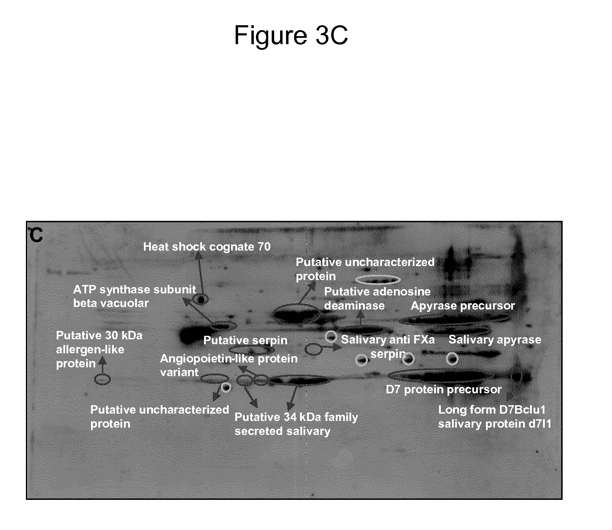

- **Figure 3****.** Immunogenic proteins of *Ae. aegyti.* 2-DE followed by Western blot of BF salivary gland extracts of *Ae. aegypti* was carried out on Immobiline DryStrips 11 cm, pH 3-10. (A) The gels were stained with PageBlue protein staining, and (B) IgG responsive proteins on PVDF membrane were detected with the pool of ten DHF patients' sera. (C) The immunoblot was then stripped and reblotted with the pool of ten *Ae. aegypti* bitten and non-infected individuals'sera. Green circles represent proteins that are not immunogenics in DHF patients. Similar results were observed in two separate experiments.

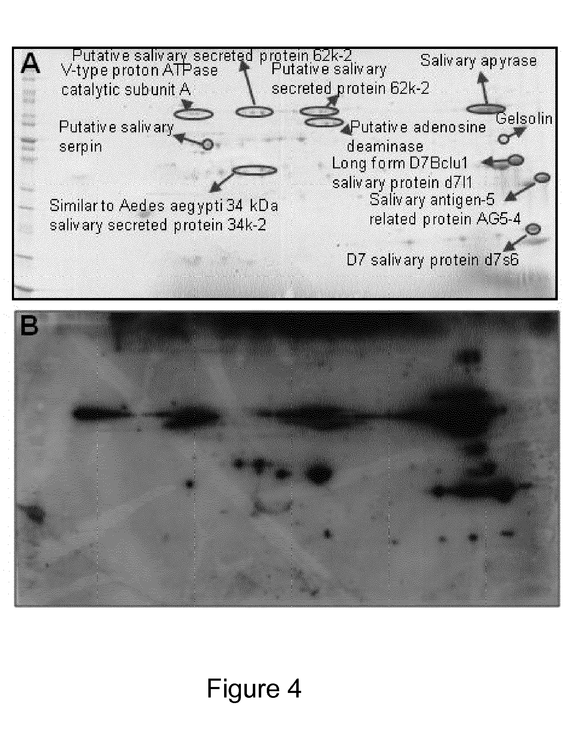

- **Figure 4****.** Immunogenic proteins of *Ae.albopictus.* Two dimentional electrophoresis follow by western blot of salivary gland extracts of *Ae. albopictus* was processed on immobiline DryStrips 11 cm, pH 3-11. The gels were stained with PageBlue™ protein staining (A) and IgG responsed proteins on PVDF membrane were detected with the pool of DHF patients' sera (B).

**Figure S1.** 2-DE profile of a pooled sample of SF and BF *Ae. aegypti* SG (50 µg of each group). The gel was stained with PageBlue™ protein staining then cored, and proteins were identified by mass spectrometry. Spot number corresponds to data presented in Table S1.

### I-Materials and methods

### - Mosquitoes and protein sample preparation

*Ae. aegypti* female mosquitoes (Liverpool strain) were reared and maintained at 26°C ± 0.5°C with 75-80% relative humidity and a 12:12 h (light:dark) photoperiod.

For female mosquitoes, MG were dissected at 3 and 10 days after feeding in order to have early and late physiological status of the midgut. Previous studies have demonstrated that DV infection was detected in MG epithelial cells at 2 Days Post Infection (DPI) and viral titers then increased until 10 DPI [12]. SG were sampled 10 days after feeding, a time frame corresponding to the extrinsic incubation period of DV, i.e., the time from initial infection of the mosquito to transmission.

In addition, Liverpool mosquitoes SG viral titer was detected at 10 DPI.

For the two groups of *Ae. aegypti* female : (i) fed exclusively on 10% sugar, i.e., "control" (SF) and (ii) fed with defibrinated human blood, the experimental group (BF), MG and SG were dissected in PBS and the tissues were subjected to three freeze-thaw cycles (liquid nitrogen) then centrifuged for 45 min at 4°C and 13,000 rpm before collection of the supernatant. Protein extracts were then desalted using a 2-D Clean-Up Kit (GE Healthcare, Germany). The Pellet was resuspended in IEF buffer (7 M urea, 2 M thiourea, 4% CHAPS, 0.8% IPG buffer, 0.2% Tergitol, 100 mM DTT) for 2D-PAGE or DIGE buffer (7 M urea, 2 M thiourea, 4% CHAPS, 0.5% Triton X 100, and 40 mM Tris-HCl) for 2D-DIGE. Protein concentrations were measured using a 2-D Quant kit (GE Healthcare, Germany).

### - Protein map of Ae. aegypti MG and SG

2-DE was carried out with 100 µg of *Ae. aegypti* MG or SG protein extracts on 18 cm Immobiline™ DryStrips pH 3-11 non-linear (NL) (GE Healthcare, Germany). Strips were rehydrated for 10-12 h at 20°C with protein samples made up to 350 µL by adding IEF buffer 1.22% DeStreak (GE Healthcare, Germany). Run conditions were as follows : temperature at 20°C; current at 50 µA per strip; 300 V (gradient) for 5 min; 300 V (step) for 30 min; 5000 V (gradient) for 3 h and then 5000 V steps up to 60,000 V·h. 2-DE was performed on 12% SDS-PAGE gel at 40 V for 45 min and then at 45 mA/gel until the bromophenol blue front had reached the end of the gel.

### - 2D-DIGE, image scanning, and statistical analysis

For each sample, 40 mosquitoes were dissected for SG and MG in triplicate from which 30 µg of proteins were used. For analytical 2D-DIGE, control and treated protein samples were compared using the CyDye™ DIGE Fluors for Ettan DIGE (Cy2, Cy3, and Cy5). Proteins were labeled according to the Ettan DIGE minimal labeling protocol (Ettan DIGE User Manual, GE Healthcare). For each sample, 20 µg of protein was labeled with 150 pmol of CyDye. An equal amount of each sample was labeled with Cy2 and added to each gel as an internal standard. The labeled samples were then pooled (according to the experimental design) and the sample volume was made up to 450 µL by adding rehydration buffer (7 M urea, 2 M thiourea, 4% CHAPS, 0.5% Triton X 100, 40 mM Tris-HCl, 1% IPG buffer and 1.2% DeStreak) prior to separation by IEF. IEF was performed with 24 cm Immobiline™ DryStrip, pH 3-10 NL. The run conditions were as followed: rehydration for 14 h at 20°C; current 50 µA per strip; 60 V (step) for 3 h; 1000 V (gradient) for 4 h; 8000 V (gradient) for 4 h; and 8000 V (step) until reaching total 64,000 V·h. 2-DE was performed on 12% SDS-PAGE gel at 15 mA/gel for 6 h and then at 30 mA/gel until the bromophenol blue front reached the end of the gel. Gels were scanned using a Typhoon™ 9400 imager (Amersham Biosciences). All gel images were acquired at 100 µm pixel resolution under nonsaturating conditions. 2D-DIGE images were analyzed using Progenesis SameSpots 3.1 software. Statistical analysis and protein quantification were carried out using the same software with a cut-off of 1.8 fold up- or down-regulated and ANOVA (*p*) < 0.05. Protein spots with a significant altered expression (p < 0.05) were trypsin digested and identified with MS.

### -Protein identification by MALDI-TOF MS

For 2D-PAGE protein map, the gels were stained with PageBlue™ protein Staining Solution overnight. After washes, visible spots were then excised manually in a laminar flow hood. For 2-D DIGE, two 2-DE gels were run with 100 µg of a mix of protein from the different samples for the MG and SG, and stained with CBB. Spots of interest in MG and SG were localized on the gel by comparing the CBB-stained spot pattern with the 2-D DIGE protein pattern. Spots were then excised. Enzymatic in-gel digestion and peptide spotting was performed automatically (Tecan freedom evo® proteomics) as previously described (1). Briefly, protein spots were digested using 150 ng of trypsin, peptide extraction was performed using 5 sonication cycles of 2 min each and peptides were concentrated 1 hour at 50°C. 0.5 µl of sample peptide and 0.5 µl of alpha-cyano-4-hydroxy-*trans*-cinnamic acid were deposited on a 384-well MALDI anchorship target using the dry-droplet procedure. Peptide sample were then desalted on target using a 10 mM phosphate buffer. Analyses were performed using an UltraFlex I MALDI TOF-TOF mass spectrometer (Bruker Daltonics, Bremen, Germany) in the reflectron mode with a 26 kV accelerating voltage and a 50 ns delayed extraction. Mass spectra were acquired manually or in the automatic mode using the AutoXecute™ module of Flexcontrol™ (Bruker Daltonics) (laser power ranged from 30 to 50%, 600 shots). Spectra were analyzed using FlexAnalysis™ software (Bruker Daltonics) and calibrated internally with the autoproteolysis peptides of trypsin (m/z 842,51 ; 1045,56 ; 2211,10). Peptides were selected in the mass range of 900-3000 Da. Peptide Mass Fingerprint identification of proteins was performed by searching against the Insecta entries of either SwissProt or TrEMBL databases (http://www.expasy.ch) and by using the Mascot v 2.2 algorithm (http://www.matrixscience.com) with trypsin enzyme specificity and one trypsin missed cleavage (2). Carbamidomethyl was set as fixed cystein modification and oxidation was set as variable methionine modification for searches. A mass tolerance of 50 ppm was allowed for identification. Matching peptides with one missed cleavage were considered as pertinent when there were two consecutives basic residues or when arginine and lysine residues were in an acidic context. Mascot scores higher than 65 were considered as significant (p < 0.05) for SwissProt and TrEMBL databases interrogation.

### - Salivary gland immunolabeling

Ten day BF and SF SGs of *Ae. aegypti* females were dissected in PBS and fixed in 4% paraformaldehyde, PBS pH 7.4 for 1 h. The tissue was then blocked for 30 min in 10% goat serum 0.3% Triton X-100 and incubated with Tetramethyl Rhodamine IsoThiocyanate (TRITC) -phalloidin for 2 h at room temperature. Hoechst dye was used to visualize the nucleus. Preparations were examined with a confocal microscope (Zeiss 5 Live Duo).

### - Immunogenicity test

SG proteins (80 µg protein) from BF mosquitoes were loaded on two Immobiline DryStrips (11 cm, pH 3-10 NL). Each sample was run in duplicate: one was stained with colloidal blue while the other was transferred to a PVDF membrane for Western blotting. The membrane was blocked in TBS Tween (0.1%, v/v), milk (5%, v/v) and incubated with a_pool of serum from ten patients with DHF. An alkaline phosphatase-conjugated monoclonal mouse anti-human IgG was used as secondary antibody and detected using the Lumi-Phos™ WB Chemiluminescent substrate (Pierce). For reblotting with pooled sera from bitten and non-infected individuals, membranes were stripped as previously reported (3).

### II - Results and discussion

### - Protein map of MG and SG on 2D-PAGE

The separation and identification of 10 days post feeding *Ae. aegypti* SF and BF pooled proteins revealed 220 MG protein spots and 102 SG protein spots for a p*I* scale of 3-11 and a Mw scale of 15-278 kDa.

Proteins from each organ were classified according to function in the MG and SG (Table S1, and Fig. S1, supporting information only concern the results obtained with SG).

Almost all of the characterized MG proteins were classified as housekeeping types, i.e. cytoskeletal proteins; proteins involved in protein modification machinery, transcription machinery, and translation machinery; transcription factors; ribosomal proteins; proteasome machinery and proteolysis proteins; transporters, and many proteins implicated in energy generation and signal transduction.

Proteins involved in metabolism were also characterized, i.e., in oxidant metabolism, carbohydrate metabolism, amino acid metabolism, lipid metabolism, and transport or intermediate metabolism.

Two secreted protease inhibitors (serpins) were recognized: Q0IEW2_AEDAE and Q0IEW4_AEDAE.

One phosphatidylethanolamine binding protein was identified as a secreted carrier-like protein belonging to the D7 family.

One enzyme involved in sugar digestion (alpha-amylase), one immunity-related protein (galectin); and 16 secreted and nonsecreted proteins with unknown function were also detected in the MG.

Similar types of housekeeping genes were expressed in the two organs, except that transcription factors, ribosomal proteins, proteasome elements, proteases, and proteins involved in signal transduction were not identified in SGs. However, this does not necessarily mean that these proteins were entirely nonexpressed in the SG; it is possible that differential expression levels exist in the two organs and there was a failure to detect the proteins in the SG.

Other groups of proteins were also identified in the SG such as secreted carrier-like proteins including D7 family, serpins, enzymes, immunity-related proteins, and proteins with unknown function.

Furthermore, *Aedes*-specific proteins and proteins with unknown function found in hematophagous dipteran saliva were also detected on the SG 2-DE gels.

In addition to a number of major, previously characterized SG proteins (D7 family, apyrase, serpin family, angiopoietin, antigen 5 protein, 30 kDa protein, F0F1 ATPase subunit, protein disulfide isomerase, adenosine deaminase, amylase, HSP70, and actin), many other proteins that have never been characterized on 2-DE analysis of SG were detected: various proteins belonging to the apyrase, serpin, and D7 families as well as housekeeping proteins such as aconitase, paramyosin, saccharopine dehydrogenase, carbonic anhydrase, and myosin heavy chain.

Spot numbers in Table S1 correspond to the SG proteins shown in Fig. S1.

Among the identified proteins, four of them were recognized as being from another *Aedes* species (*Ae. albopictus*): (i) long form D7 salivary protein D712 (Q5MIW6_AEDAL), (ii) long form D7Bclu1 salivary protein d711 (QSMIW7_AEDAL), (iii) salivary apyrase (Q5MIX3_AEDAL), and (iv) serpin protein (Q5MIW0_AEDAL). Such homologies would be expected between two mosquitoes of the same subgenus.

The differential expression of protein by 2D-DIGE in each organ with regard to diet was investigated for a comprehensive understanding of the normal MG and SG proteomes could be useful when it comes to pathogenesis.

### - Differential protein expression between SF and BF groups in MG and SG

These results extend the understanding of the 2D profile of salivary proteins and provide the first comparison by 2D-DIGE of the level of SF and BF protein expression in female *Ae. aegypti* mosquitoes at 3 and 10 days post feeding for MG and 10 days for SG.

After detection of protein spots, image gels were "cleaned off" for speckling and artefact spots. In total, 2000 spots were detected on the MG gel (Fig. 1A) and 706 on the SG gel (Fig. 1B).

The MG protein profile is more complex than the SG one, considering the number of proteins present in each organ (see Fig. 1). One of the most important parameters in the 2-DE technique is the quantity of proteins loaded onto each gel (which must be the same). There may have been some differences but not in these p*I* and Mw ranges. It is also well known that the molecular weight of proteins detected in 2-DE experiments is generally limited to the range of 150-15 kDa so very high and low molecular weight proteins as well as peptides are missed.

However, in the SG, 84 protein spots were detected as being differentially expressed: 37 spots were denser in the BF insects and 47 spots were weaker (Fig. 1B). The absence of any difference in global protein profile suggests that all these proteins are normally expressed in each organ but the nature of the ingested meal can modulate their expression levels.

A total of 32 protein spots were characterized in *Ae. aegypti* SG: 17 were up-regulated and 15 down-regulated in the BF group compared to the SF group (Table 1, Fig. 1B). From the 17 spots that were up-regulated, 13 proteins could be identified (see Table 1). Among the up-regulated SG proteins, three D7 proteins were highly expressed in BF SG: long form D7 salivary protein D712 (Q5MIW6_AEDAL), D7 protein, and putative (Q0IF93_AEDAE) and D7 protein precursor (D7_AEDAE).

The long form D7 salivary protein D712 is one type of *Ae. albopictus* D7 protein that is best matched with the 37 kDa salivary gland allergen Aed a 2 (D7 protein precursor: ALL2_AEDAE) of *Ae. aegypti* D7 protein.

The D7 protein family is related to odorant binding proteins that are able to bind host biogenic amine to antagonize vasoconstictor, platelet-aggregating, and pain-inducing properties. Additionally, the long form D7Bclu1 salivary protein d711 (*Ae. albopictus* protein), also identified in the protein map of the invention, matches best with a putative D7 protein, Q0IF93_AEDAE. These similarities were discussed above in the part of protein map. Aldehyde dehydrogenase possesses oxidoreductase activity that catalyzes the NAD⁺-dependent oxidation of aldehyde to carboxylic acid, and this ubiquitous enzyme is related to energy metabolism.

Adenosine deaminase was also up-regulated in SG after a blood meal. This enzyme hydrolyzes adenosine to inosine and ammonia, and the removal of adenosine may inhibit platelet aggregation and mast cell degranulation. Inosine interferes with host coagulation at the bite site to inhibit the production of inflammatory cytokines. All these processes may enhance blood feeding.

Salivary serpin putative anticoagulant (Q5MIW0_AEDAL: *Ae. albopictus-specific* protein) was expressed 3.5 times more in BF SG than SF SG. The serpin superfamily is reported to inhibit endogenous proteases like the serine proteases involved in regulating coagulation, such as thrombin and factor Xa.

In addition, a serine protease cascade is one innate insect immune response which activates prophenoloxidase in plasma. (*Drosophila* serpins have been shown to be inhibitory and involved in regulating Toll and prophenoloxidase signaling pathways). The up-regulation of salivary apyrase and its precursor in BF SG was also observed. Two spots of differentially expressed salivary apyrase (Q5MIX3_AEDAL) were identified as *Ae. albopictus* specific: they were found at levels 2-3.1 times higher in BF SG compared to SF one.

Apyrase is an enzyme that inhibits ADP-dependent platelet aggregation during blood-feeding and prevents neutrophil activation. Apyrase is synthesized in the adult female SG and accumulates in the distal lateral lobes.

A putative 30 kDa allergen-like protein (accession number UniProtKB/TrEMBL: Q8T9T8_AEDAE) previously identified as a 30 kDa antigen (gi: 18568322) by Ribeiro *et al. (4),* a species of unknown function, is ubiquitously found in the SGs of adult mosquitoes. Studying saliva allergens could provide valuable information on immune responses, with a view to developing new diagnostic tests for allergies to mosquito bites.

Four secreted proteins were identified: Q8T9V0_AEDAE, which binds a receptor involved in signal transduction; a 19.6 kDa secreted protein precursor (Q58HB7_AEDAE); a putative secreted protein (Q8T9W0_AEDAE); and a putative uncharacterized protein (Q17NC3_AEDAE). The functions of the last three proteins are unknown.

Almost all the proteins that were up-regulated in BF SG seem to have an important role in successful blood feeding.

The proteins that were down-regulated in BF group (up-regulated in SF mosquitoes) tended to have housekeeping functions (Table 1): paramyosin, long form and actin-1 are cytoskeletal proteins. Paramyosin is a component of the thick filament core-regulating complex of invertebrate muscle. Actin, the main protein of muscle and cytoskeleton, is the major component of thin filament in muscle. It is also one of three subclasses of the cytoskeleton (microfilament, intermediate filament, and microtubule) found in the cytoplasm of all cells. It plays important roles in cellular dynamics such as the maintenance of cell shape, cell growth, cellular motion, intracellular transport, and cell division and differentiation. Differential actin expression of actin was also studied by immunofluorescence (IF) and will be discussed below in detail in the section of IF assay.

Many proteins that were up-regulated in the SG of SF *Ae. aegypti* have a function in metabolism, ion transport, and energy generation. Mitochondrial porin and ATP synthase subunit beta are housekeeping products involved in anion and hydrogen ion transport, respectively. Porins are major channel-forming proteins found in the mitrochondrial outer membrane. All metabolites as well as ATP and ADP pass through these channels.

Mitochondrial aconitase, mitochondrial ATP synthase β chain, NADH-ubiquinone oxidoreductase 75 kDa subunit, ubiquinol-cytochrome c reductase complex, pyruvate kinase, and probable citrate synthase 1 and 2 are enzymes involved in the cell's energy-generating machinery. Oxidative phosphorylation occurs at the inner membrane of the mitochondrion depending on the relative proportions of NADH, oxygen, ATP, ADP, and inorganic phosphate. Both pyruvate kinase and citrate synthase are involved in glycolysis and the tricarboxylic acid cycle. Saccharopine dehydrogenase plays a role in conversion of lysine to glutamate. Two enzymes of lysine catabolism are synthesized from the lysine-ketoglutarate reductase/saccharopine dehydrogenase (LKR/SDH) gene and this bifunctional enzyme is conserved from plants through animals. Acyl-CoA dehydrogenase catalyzes the initial mitochondrial step in the β-oxidation fatty acid cycle. Apart from pyruvate kinase, all these proteins are mitochondria-specific and their preferential expression in SF mosquitoes points to their role in basic metabolism. One uncharacterized protein (Q16QU2_AEDAE) of unknown function showed highly differential protein expression.

Two groups of proteins were distinct: those proteins that were up-regulated in SF mosquitoes seemed to be specific to the maintenance of physiological functions, whereas those that were up-regulated in BF mosquitoes were proteins that facilitate the process of blood-feeding. All those sequences that up-regulated in BF mosquitoes contained a cleavage site meaning that they were secreted proteins in saliva. This characteristic confirms their important role in facilitating blood feeding when the mosquito takes a blood meal. It is believed by the inventors that the production of these molecules is strongly stimulated after mosquitoes have had their first blood meal because they are only weakly expressed when mosquitoes are feeding exclusively on sugar.

### - Immunofluorescence assay (IF)

As described above in the section of differential protein expression between SF and BF groups in MG and SG, actin and paramyosin were more highly expressed in SF SG (Table 1). These proteins are constituents of the cytoskeleton and muscle fibers. The disruption of cytoskeletal links leads to changes in cell shape.
Blood-feeding probably modifies the cytoskeleton and/or the shape of SG cells. For these reasons, SG morphology was checked by IF. Whole SG from BF and SF mosquitoes were dissected out, actin expression was labeled using TRITC-phalloidin, and nuclei were stained with Hoechst dye (Fig. 2). Actin seemed to localize to the cell boundary and along the duct of each lobe in both SF and BF SG. Nuclei were located apically. No difference was observed between the two SG groups morphology, condition, or cytoskeletal organization. These results suggest that after a blood meal there is no disruption of the SG cytoskeletal.

### - Immunogenic proteins

The major risk of DHF is exacerbation to fatal DSS. The major pathophysiologic hallmarks that determine disease severity and distinguish DHF from dengue fever are plasma leakage (as a result of increased vascular permeability) and abnormal coagulation. The mechanisms that lead to DHF/DSS are a subject of intense debate. The possible role of mosquito saliva in these mechanisms has never been evaluated. Remoue et al. ( Transactions of the Royal Society of Tropical Medicine and Hygiene, 2006, vol. 100, 363-370) demonstrated that some salivary proteins are immunogenic and can initiate a specific antibody response. In addition, the anti-saliva antibody response is a reliable marker of exposure to vector-borne diseases in individuals exposed to bites of arthropod vectors. As described for maxadilan, which enhances *Leishmania* infection, the SG molecules detected by serum of DHF_patients may enhance virus infection and thus constitute vaccine targets. In this study, the inventors identified for the first time the immunogenic salivary molecules of *Ae. aegypti,* with sera of DHF patients and bitten non-infected individuals (Fig. 3).

Within the BF SG proteins of *Ae. aegypti,* fifteen immunogenic proteins of sequences SEQ ID N°1-15 were identified in the study: heat shock cognate 70, ATP synthase subunit beta vacuole, putative adenosine deaminase, salivary apyrase and its precursor, putative serpin, salivary anti-FXa serpin, fibrinogen and fibronectin, angiopoietin-like protein variant, putative secreted salivary 34 kDa family, D7 protein precursor, long form D7Bclu1 salivary protein d711, and two putative uncharacterized proteins (62kDa, Q17NC2_AEDAE and Q17F11_AEDAE).

These results show that there is a strong immune response to *Ae. aegypti* salivary proteins.

The above results present the first differentially expressed *Aedes* SG proteins analysis by 2D-DIGE relative to blood feeding. Additionally, the protein map of *Ae. aegypti* MG and SG was completed. Moreoser, for the first time, identified *Ae. aegypti* saliva proteins exhibiting immunogenic properties in *Ae. aegypti*-bitten individuals were identified and characterisez. These proteins play a role in dengue virus infection or are epidemiological indicators of exposure to dengue. Understanding the interactions between vectors and vertebrate hosts will promote the development of effective and novel control strategies for this major human emerging disease.

**Table 1. Differential protein expression between SF and BF groups in Ae. aegypti salivary gland.**

| **Spot number** | **ANOVA (*p-*value)** | **X-food average** | **ANV^{a)}** | | **Accession no.** | **Name of protein^{b)}** | **MASCOT score^{c)}** | **Coverage (%)** |
|---|---|---|---|---|---|---|---|---|
| | | | **Sugar** | **Blood** | | | | |
| Up-regulated | | | | | | | | |
| 1 | 0.002 | 8.1 | 0.188 | 1.528 | Q5MIW6_AEDAL | Long form D7 salivary protein D712 | 73 | 22 |
| 5 | 9.96E-04 | 3.7 | 0.46 | 1.715 | Q16P57_AEDAE | Aldehyde dehydrogenase | 85 | 18 |
| 6 | 0.003 | 3.5 | 0.461 | 1.626 | Q5MIW0_AEDAL | Salivary serpin putative anticoagulant | 98 | 24 |
| 8 | 0.001 | 3.1 | 0.45 | 1.375 | Q5MIX3_AEDAL | Salivary apyrase | 142 | 30 |
| 10 | 0.002 | 2.9 | 0.506 | 1.487 | APY_AEDAE | Apyrase precursor | 260 | 48 |
| 13 | 5.89E-04 | 2.7 | 0.595 | 1.602 | Q0IF93_AEDAE | D7 protein, putative | 69 | 17 |
| 18 | 1.66E-04 | 2.2 | 0.762 | 1.701 | D7_AEDAE | D7 protein precursor | 83 | 30 |
| 19 | 4.43E-04 | 2.2 | 0.651 | 1.435 | APY_AEDAE | Apyrase precursor | 253 | 42 |
| 22 | 0.002 | 2.1 | 0.694 | 1.439 | QI7NC3_AEDAE | Putative uncharacterized protein | 203 | 40 |
| 23 | 0.008 | 2.1 | 0.966 | 1.987 | Q58HB7_AEDAE | Putative 19.6 kDa secreted protein precursor | 90 | 23 |
| 24 | 8.04E-04 | 2.0 | 0.693 | 1.395 | Q179D4_AEDAE | Adenosine deaminase | 142 | 31 |
| 25 | 0.004 | 2.0 | 0.635 | 1.254 | Q8T9W0_AEDAE | Putative secreted protein | 59 | 27 |
| 26 | 0.006 | 2.0 | 0.657 | 1.282 | Q5MIX3_AEDAL | Salivary apyrase | 152 | 31 |
| 27 | 6.71E-05 | 1.9 | 0.792 | 1.53 | Q8T9V0_AEDAE | Putative secreted protein | 76 | 23 |
| 28 | 0.009 | 1.9 | 0.91 | 1.685 | Q8T9T8_AEDAE | Putative 30 kDa allergen-like protein (putative uncharacterized protein) | 81 | 28 |
| 29 | 6.16E-04 | 1.8 | 0.749 | 1.334 | Q179D4_AEDAE | Adenosine deaminase | 159 | 29 |
| 30 | 0.019 | 1.8 | 0.904 | 1.591 | Q58HB7_AEDAE | Putative 19.6 kDa secreted protein precursor | 90 | 23 |

| Down-regulated | | | | | | | | |
|---|---|---|---|---|---|---|---|---|
| 2 | 0.009 | 7.5 | 2.482 | 0.331 | Q16QU2_AEDAE | Putative uncharacterized protein | 72 | 23 |
| 3 | 7.05E-05 | 6.5 | 2.124 | 0.327 | Q16RF4_AEDAE | Paramyosin, long form | 169 | 20 |
| 4 | 7.28E-04 | 4.5 | 2.181 | 0.49 | ACT1_AEDAE | Actin-1 | 57 | 21 |
| 7 | 4.91E-04 | 3.2 | 1.975 | 0.617 | Q16LR5_AEDAE | NADH-ubiquinone oxidoreductase 75 kDa subunit | 149 | 23 |
| 9 | 0.003 | 3.0 | 1.772 | 0.582 | Q16KR4_AEDAE | Aconitase, mitochondrial | 255 | 35 |
| 11 | 0.004 | 2.9 | 1.617 | 0.558 | Q179J9_AEDAE | Mitochondrial ATP synthase b chain | 70 | 33 |
| 12 | 0.002 | 2.9 | 1.795 | 0.622 | Q1HR57_AEDAE | Mitochondrial porin | 160 | 42 |
| 14 | 0.012 | 2.4 | 1.698 | 0.718 | Q16KR4_AEDAE | Aconitase, mitochondrial | 255 | 35 |
| IS | 0.001 | 2.3 | 1.707 | 0.749 | Q17AK0_AEDAE | Ubiquinol-cytochrome c reductase complex core protein | 131 | 34 |
| 16 | 0.011 | 2.3 | 1.631 | 0.72 | Q16KR4_AEDAE | Aconitase, mitochondrial | 255 | 35 |
| 17 | 0.003 | 2.2 | 1.68 | 0.748 | Q17FL3_AEDAE | ATP synthase subunit beta | 266 | 45 |
| 20 | 0.008 | 2.1 | 1.675 | 0.781 | Q17GM7 CISY1_AEDAE | Probable citrate synthase 1, mitochondria | 136 | 35 |
| | | | | | Q16P20 GISY2_AEDAE | Probable citrate synthase 2, mitochondrial precursor | 134 | 35 |
| 21 | 0.003 | 2.1 | 1.555 | 0.746 | Q16LP4_AEDAE | Pyruvate kinase | 123 | 22 |
| 31 | 8.18E-04 | 1.8 | 1.38 | 0.786 | Q16FJ9_AEDAE | Saccharopine dehydrogenase | 127 | 19 |
| 32 | 0.002 | 1.8 | 1.467 | 0.837 | Q16GA1_AEDAE | Acyl-CoA dehydrogenase | 202 | 40 |

| | | | | | | | | |
|---|---|---|---|---|---|---|---|---|
| a) Average normalized volume. expasy.org/). b) Accession number in SwissProt and TrEMBL protein databases (http://www.expasy.org/). c) MASCOT scores >50 indicate significant identity or extensive homology. | | | | | | | | |

**Table S1: Salivary gland proteins identified by 2D-PAGE and MALDI-TOF. Database searches used the MASCOT program and SwissProt/TrEMBL databases. Molecular mass, pI, MASCOT score (Sc) for the best match and covered sequence (cv) are shown**

| Identification | SwissProt/TrEMBL | Spot no. | Accession no. | Mass (Da) | pI | Sc | cv | Comments/biological activity | Subcellular localization/cellular component | Similarity |
|---|---|---|---|---|---|---|---|---|---|---|
| **Secreted carrier-like proteins including D7 family** | | | | | | | | | | |
| D7 protein, putative - *Aedes aegypti* | Q0IF93_AEDAE | 109 | gi\|122115019 | 38603.0 | 9.32 | 69 | 17 | Odorant binding, transport | | |
| Long form D7 salivary protein D7I2 - *Aedes albopictus* | Q5MIW6_AEDAL | 114 | gi\|74767432 | 36883.0 | 8.60 | 73 | 22 | Odorant binding, transport | | |
| D7 protein precursor - *Aedes aegypti* | ALL2_AEDAE | 101, 102,103, 105, 107 | gi\|205525919 | 37005 | 8.36 | 83 | 30 | Thought to be involved in blood-feeding | Secreted, expressed in the distal-lateral and medial lobes of the adult female salivary gland | Two named isoforms (FASTA) produced by alternative splicing |
| Long form D7Bclu1 salivary protein d7I1 - *Aedes albopictus* | Q5MIW7_AEDAL | 108 | gi\|74767433 | 38404 | 8.87 | 64 | 22 | Transport, odorant binding | | |
| **Secreted protease inhibitors** | | | | | | | | | | |
| **Serpins** | | | | | | | | | | |
| Serine protease inhibitor (serpin-4) putative - *Aedes aegypti* | Q17HD9_AEDAE | 66, 70 | gi\|122118433 | 47752 | 6.27 | 141 | 35 | Peptidase activity, serine-type endopeptidase inhibitor activity | | Belongs to the serpin family. |
| Serine protease inhibitor - *Aedes aegypti* | Q17HD8_AEDAE | 70 | gi\|122118432 | 41802.0 | 7.55 | 93 | 23 | Peptidase activity, serine-type endopeptidase inhibitor activity | | |
| Salivary anti FXa serpin - *Aedes aegypti* | Q1HRU9_AEDAE | 67 | gi\|121959442 | 47873.0 | 6.13 | 84 | 24 | Serine-type endopeptidase inhibitor activity | | Belongs to the serpin family . |
| Salivary serpin putative anticoagulant - *Aedes albopictus* | Q5MIW0_AEDAL | 65 | gi\|74903324 | 46739 | 6.15 | 98 | 24 | Serine-type endopeptidase inhibitor activity | | Belongs to the serpin family. |
| SERPIN1 protein, | Q17G63 AEDAE | 72, 73, 74, | gi\|122106702 | 47009 | 5.41 | 290 | 43 | Serine-type | | Belongs to the |
| putative - *Aedes aegypti* | | 75, 77, 78 | | | | | | inhibitor activity | | serpin family |
| Putative serpin - *Aedes aegypti* | Q8T9U7_AEDAE | 73, 74 | gi\|74934477 | 47087.0 | 5.18 | 249 | 40 | Serine-type endopeptidase inhibitor activity | | Belongs to the serpin family |
| **Enzyme** | | | | | | | | | | |
| **Nucleotidases** | | | | | | | | | | |
| Salivary apyrase - *Aedes albopictus* | Q5MIX3_AEDAL | 40 | gi\|74837243 | 62760.0 | 8.66 | 152 | 31 | Hydrolase activity, acting on ester bonds, metal ion binding, nucleotide binding, nucleotide catabolic process | Secreted | Belongs to the 5'-nucleotidase family |
| Salivary apyrase, putative - *Aedes aegypti* | Q176L8_AEDAE | 34,35 | gi\|122106133 | 63199 | 8.65 | 93 | 16 | Hydrolase activity, nucleotide catabolic process | Secreted | Belongs to the 5'-nucleotidase family |
| Apyrase precursor - *Aedes aegypti* | APY_AEDAE | 29, 30, 31, 32,33 | gi\|193806340 | 62691.0 | 8.47 | 260 | 48 | Facilitates hematophagy by preventing ADP-dependent platelet aggregation in the host. May reduce probing time by facilitating the speed of locating blood. | Secreted, salivary gland specific | Belongs to the 5'-nucleotidase family. |
| Adenosine deaminase - *Aedes aegypti* | Q179D4_AEDAE | 37 | gi\|122094769 | 59834 | 6.37 | 142 | 31 | Deaminase activity, purine ribonucleoside monophosphate biosynthetic process | | |
| Putative adenosine deaminase - *Aedes aegypti* | Q8T9T6_AEDAE | 38, 39, 43, 44 | gi\|74819780 | 60525 | 6.51 | 227 | 50 | Deaminase activity, purine ribonucleoside monophosphate biosynthetic process | | |
| Inosine-uridine preferring nucleoside hydrolase - *Aedes aegypti* | Q175Y5_AEDAE | 95 | gi\|122106110 | 37911 | 4.93 | 82 | 23 | Hydrolase activity | | |
| Sugar digestion related | | | | | | | | | | |
| Alpha-amylase I precursor - *Aedes aegypti* | AMY1_AEDAE | 4, 6 | gi\|205638829 | 80937 | 6.41 | 112 | 21 | Alpha-amylase activity | Expressed specifically in the proximal-lateral lobes of the adult female salivary gland. | Belongs to the glycosyl hydrolase 13 family |
| **Immunity related proteins** | | | | | | | | | | |

| **Angiopoietin** | | | | | | | | | | |
|---|---|---|---|---|---|---|---|---|---|---|
| Angiopoietin-like protein variant - *Aedes aegypti* | Q1HRV2_AEDAE | 60, 99, 104 | gi\|121959445 | 33401 | 5.74 | 139 | 38 | Receptor binding, signal transduction | | Contains 1 fibrinogen C-terminal domain |
| Fibrinogen and fibronectin - *Aedes aegypti* | Q17NC0_AEDAE | 80, 91 | gi\|122095637 | 33611 | 6.04 | 96 | 31 | Receptor binding, signal transduction | | Contains 1 fibrinogen C-terminal domain. |
| Putative secreted protein - *Aedes aegypti* | Q8T9V0_AEDAE | 85 | gi\|74819788 | 33698.0 | 5.12 | 76 | 23 | Receptor binding, signal transduction | | Contains 1 fibrinogen C-terminal domain |
| Putative uncharacterized protein - *Aedes aegypti* | Q17NB9_AEDAE | 98 | gi\|122107139 | 33361.0 | 5.74 | 89 | 31 | Receptor binding, signal transduction | | Contains 1 fibrinogen C-terminal domain. |

| **Housekeeping product** | | | | | | | | | | |
|---|---|---|---|---|---|---|---|---|---|---|
| **Transporters** | | | | | | | | | | |
| Vacuolar ATP synthase catalytic subunit A - *Aedes aegypti* | VATA_AEDAE | 25 | gi\|145559538 | 68186 | 5.26 | 237 | 38 | Catalytic subunit of the peripheral V1 complex of vacuolar ATPase. V-ATPase vacuolar ATPase is responsible for acidifying a variety of intracellular compartments in eukaryotic cells. | V-ATPase is an heteromultimeric enzyme composed of a peripheral catalytic V1 complex (main components: subunits A, B, C, D, E, and F) attached to an integral membrane V0 proton pore complex (main component: the proteolipid protein). | Belongs to the ATPase alpha/beta chains family |
| Vacuolar ATPase B subunit - *Aedes aegypti* | Q9XYC8_AEDAE | 55 | gi\|74949759 | 55181 | 5.38 | 80 | 20 | Hydrogen ion transporting ATP synthase activity, rotational mechanism, hydrogen ion transporting ATPase activity, hydrogen-exporting ATPase activity, phosphorylative mechanism, ATP metabolic process, energy coupled proton transport, against electrochemical gradient | Cytoplasm, proton-transporting two-sector ATPase complex | Belongs to the ATPase alpha/beta chains family. |
| Carbonic anhydrase-*Aedes aegypti* | Q17BL7_AEDAE | 119 | gi\|122094901 | 31287 | 5.73 | 92 | 33 | Carbonate dehydratase activity, zinc ion binding, one-carbon compound metabolic process | | |
| Mitochondrial porin (voltage-dependent anion channel, voltage-dependent anion-selective channel) *Aedes aegypti* | Q1HR57_AEDAE | 121, 122 | gi\|121959252 | 30677 | 8.63 | 160 | 42 | Voltage-gated anion channel activity, anion transport | Integral to membrane, mitochondrial outer membrane | |
| ATP synthase subunit beta - *Aedes aegypti* | Q1HR61_AEDAE | 57 | gi\|121959256 | 53937.0 | 5.03 | 89 | 29 | Produces ATP from ADP in the presence of a proton gradient across the membrane, ATP binding, hydrogen ion transporting ATP synthase activity, rotational mechanism, hydrogen ion transporting ATPase activity, hydrogen-exporting ATPase activity, phosphorylative mechanism, metal ion binding, ATP synthesis coupled proton transport | Integral to membrane, proton-transporting ATP synthase complex, catalytic core F(1) | Belongs to the ATPase alpha/beta chains family. |
| ATP synthase subunit beta - *Aedes aegypti* | Q17FL3_AEDAE | 57, 58, 59 | gi\|122095158 | 53940 | 5.02 | 282 | 47 | Produces ATP from ADP in the presence of a proton gradient across the membrane. ATP binding, hydrogen ion transporting ATP synthase activity, rotational mechanism, hydrogen ion transporting ATPase activity, hydrogen-exporting ATPase activity, phosphorylative mechanism, metal ion binding, ATP synthesis coupled proton transport | Integral to membrane, proton-transporting ATP synthase complex, catalytic core F(1) | Belongs to the ATPase alpha/beta chains family. |
| Catalase - *Aedes aegypti* | Q1HRH7_AEDAE | 53 | gi\|122107851 | 48494 | 7.28 | 175 | 40 | Catalase activity, response to oxidative stress | | |
| **Carbohydrate metabolism** | | | | | | | | | | |
| Triosephosphate isomerase - *Aedes aegypti* | Q17HW3_AEDAE | 130' | gi\|122118464 | 26305.0 | 5.38 i | 113 | 41 | Triose-phosphate isomerase activity, metabolic process | Cytoplasmic | Belongs to the triosephosphate isomerase family |
| Phosphoglycerate kinase - *Aedes aegypti* | Q95UR6_AEDAE | 76 | gi\|74938314 | 43699 | 7.63 | 128 | 43 | ATP binding, phosphoglycerate kinase activity, glycolysis | | Belongs to the phosphoglycerate kinase family |
| Phosphoglycerate kinase - Aedes *aegypti* | Q8WQL0_AEDAE | 76 | gi\|74935682 | 43829.0 | 6.63 | 70 | 33 | ATP binding, phosphoglycerate kinase activity, glycolysis | Cytoplasmic | Belongs to the phosphoglycerate kinase family |
| Probable citrate synthase 1, mitochondrial - *Aedes aegypti* | CISY1_AEDAE | 90 | gi\|122095223 | 51624 | 8.91 | 136 | 35 | Citrate (Si)-synthase activity, tricarboxylic acid cycle, carbohydrate metabolic process | Mitochondrion matrix | Belongs to the citrate synthase family |
| Probable citrate synthase 2, mitochondrial precursor - *Aedes aegypti* | CISY2_AEDAE | 90 | gi\|122067459 | 51608.0 | 8.91 | 134 | 35 | Citrate (Si)-synthase activity, tricarboxylic acid cycle, carbohydrate metabolic process | Mitochondrion matrix | Belongs to the citrate synthase family |

| **Lipid metabolism** | | | | | | | | | | |
|---|---|---|---|---|---|---|---|---|---|---|
| Acyl-CoA dehydrogenase - *Aedes aegypti* | Q16GA1_AEDAE | 93 | gi\|122104662 | 45542 | 8.35 | 202 | 40 | Acyl-CoA dehydrogenase activity, electron carrier activity, FAD binding, metabolic process | | |
| Acyl-CoA dehydrogenase - *Aedes aegypti* | Q171S4_AEDAE | 96 | gi\|122117595 | 46380 | 6.05 | 52 | 12 | Acyl-CoA dehydrogenase activity, electron carrier activity, FAD binding, metabolic process | | |
| **Amino acid metabolism** | | | | | | | | | | |
| Saccharopine dehydrogenase - Aedes *aegypti* | Q16FJ9_AEDAE | 2, 3 | gi\|122116345 | 102704 | 6.37 | 127 | 19 | Binding, oxidoreductase activity, electron transport | | |
| Glutamate semialdehyde dehydrogenase - *Aedes aegypti* | Q174N2_AEDAE | 14,15 | gi\|122068317 | 86904 | 6.76 | 208 | 26 | Glutamate 5-kinase activity, glutamate-5-semialdehyde dehydrogenase activity, proline biosynthetic process | Cytoplasm | |
| Aspartate ammonia lyase - *Aedes aegypti* | Q16ZL0_AEDAE | 69 | gi\|122068091 | 54328 | 8.64 | 81 | 21 | Fumarate hydratase activity, fumarate metabolic process, tricarboxylic acid cycle | Tricarboxylic acid cycle enzyme complex | |

| **Intermediate metabolism** | | | | | | | | | | |
|---|---|---|---|---|---|---|---|---|---|---|
| Transketolase - *Aedes aegypti* | Q17CT0_AEDAE | 36 | gi\|122068739 | 67891 | 6.54 | 205 | 35 | Transferase activity, metabolic process | | |
| 4-Hydroxybutyrate CoA-transferase, putative - *Aedes aegypti* | Q0IG02_AEDAE | 56 | gi\|122115056 | 51817 | 8.15 | 147 | 34 | Transferase activity, acetyl-CoA metabolic process | | |
| Cyclohex-1-ene-1-carboxyl-CoA hydratase, putative - *Aedes aegypti* | Q17E10_AEDAE | 129 | gi\|122106558 | 31510 | 8.72 | 109 | 29 | Catalytic activity, metabolic process | | Belongs to the enoyl-CoA hydratase/isomerase family |
| Aldo-keto reductase - *Aedes aegypti* | Q17DM9_AEDAE | 100 | gi\|122106530 | 36387 | 6.56 | 88 | 28 | Oxidoreductase activity | | |
| Mandelate racemase - *Aedes aegypti* | Q17LB3_AEDAE | 61 | gi\|122107012 | 51506.0 | 5.56 | 52 | 15 | Catalytic activity, metabolic process | | |

| **Transcription machinery** | | | | | | | | | | |
|---|---|---|---|---|---|---|---|---|---|---|
| Elongation factor 1-gamma - *Aedes aegypti* | Q1HR56_AEDAE | 81, 89 | gi\|122070113 | 48953 | 6.04 | 145 | 32 | Translation elongation factor activity, translational elongation | Eukaryotic translation elongation factor 1 complex | Contains one GST C-terminal domain |
| Ebna2 binding protein P100 - *Aedes aegypti* | Q17PM3_AEDAE | 5, 6, 7, 10 | gi\|122107223 | 103314 | 7.03 | 243 | 33 | Nuclease activity, nucleic acid binding, protein binding, RNA interference | RNA-induced silencing complex | |
| Polyadenylate-binding protein - Aedes *aegypti* | Q1HR66_AEDAE | 26 | gi\|122127519 | 69679 | 9.48 | 97 | 25 | Nucleotide binding, RNA binding | | Contains one PABC domain |

| **Translation machinery** | | | | | | | | | | |
|---|---|---|---|---|---|---|---|---|---|---|
| Elongation factor 1- alpha *Aedes aegypti* | Q1HR88_AEDAE | 64 | gi\|122107849 | 50441.0 | 9.16 | 93 | 24 | Promotes the GTP- dependent binding of aminoacyl-tRNA to the A-site of ribosomes during protein biosynthesis. GTP binding, GTPase activity, translation elongation factor activity. translational elongation | Gytoplasm | Belongs to the GTP-binding elongation factor family. EF-Tu/EF-1A subfamily |
| Elongation factor 2 - *Aedes aegypti* | Q8T4S0_AEDAE | 9 | gi\|75017946 | 94280 | 5.99 | 112 | 17 | GTP binding, GTPase activity, translation elongation factor activity | | |
| Elongation factor 2 - Aedes *aegypti* | Q9BME7_AEDAE | 9 | gi\|75021733 | 94344.0 | 5.99 | 137 | 22 | GTP binding, GTPase activity, translation elongation factor activity | | |
| Elongation factor tu - *Aedes aegypti* | Q16YK5_AEDAE | 68 | gi\|122105752 | 51063 | 8.05 | 131 | 33 | Promotes the GTP-dependent binding of aminoacyl-tRNA to the A-site of ribosomes during protein biosynthesis. GTP binding, GTPase activity, translation elongation factor activity, translational elongation | Intracellular | Belongs to the GTP-binding elongation factor family. EF- Tu/EF-1A subfamily. |

| **Energy generation machinery** | | | | | | | | | | |
|---|---|---|---|---|---|---|---|---|---|---|
| Mitochondrial F1F0-ATP synthase subunit d/ATP7 - *Aedes aegypti* | Q1HR21_AEDAE | 132 | gi\|121959223 | 19545.0 | 5.26 | 63 | 31 | Hydrogen ion transporting ATP synthase activity, rotational mechanism, hydrogen ion transporting ATPase activity, ATP synthesis coupled proton transport | Proton-transporting 2-sector ATPase complex | |
| NADH-ubiquinone oxidoreductase 75 kDa subunit - *Aedes aegypti* | Q16LR5_AEDAE | 20 | gi\|122067320 | 79150 | 6.54 | 149 | 23 | Two iron, two sulfur cluster binding, four iron, four sulfur cluster binding, iron ion binding, NADH dehydrogenase (ubiquinone) activity, ATP synthesis coupled electron transport | Membrane | Belongs to the complex I 75 kDa subunit family. |
| NADH-ubiquinone oxidoreductase 39 kDa subunit - *Aedes aegypti* | Q16VZ4_AEDAE | 94 | gi\|122105600 | 46039 | 9.03 | 153 | 42 | Catalytic activity, coenzyme binding, cellular metabolic process | | |
| Ubiquinol-cytochrome c reductase complex core protein - Aedes *aegypti* | Q17AK0_AEDAE | 92 | gi\|122094838 | 45542 | 7.66 | 131 | 34 | Metalloendopeptidase activity, zinc ion binding, proteolysis | | Belongs to the peptidase M16 family |
| Aldehyde dehydrogenase - Aedes *aegypti* | Q16P57_AEDAE | 51 | gi\|122105128 | 58257 | 6.82 | 85 | 18 | Oxidoreductase activity, metabolic process | | |
| Mitochondrial ATP synthase alpha subunit - *Aedes aegypti* | Q1HRQ7_AEDAE | 62, 63 | gi\|122127537 | 59355 | 9.01 | 211 | 37 | Produces ATP from ADP in the presence of a proton gradient across the membrane. ATP binding, hydrogen ion transporting ATP synthase activity, rotational mechanism. hydrogen ion transporting ATPase activity, metal ion binding, ATP synthesis coupled oroton transport | Proton-transporting ATP synthase complex, catalytic core F(1) | Belongs to the ATPase alpha/beta chains family |
| Mitochondrial ATP synthase b chain - *Aedes aegypti* | Q179J9_AEDAE | 131 | gi\|122094781 | 27023.0 | 8.96 | 70 | 33 | Hydrogen ion transporting ATP synthase activity, rotational mechanism,hydrogen ion transporting ATPase activity, ATP synthesis coupled proton transport | Proton-transporting two-sector ATPase complex | |
| Adenylate kinase - *Aedes aegypti* | Q1HQK0_AEDAE | 125 | gi\|121959090 | 26257 | 6.54 | 101 | 35 | Adenylate kinase activity, ATP binding, nucleobase, nucleoside, nucleotide and nucleic acid metabolic process | | Belongs to the adenylate kinase family |
| Pyruvate kinase - *Aedes aegypti* | Q16LP4_AEDAE | 52 | gi\|122116721 | 55510 | 7.1 | 123 | 22 | Magnesium ion binding, potassium ion binding, pyruvate kinase activity, glycolysis | | Belongs to the pyruvate kinase family |
| Enolase - *Aedes aegypti* | Q17KK5_AEUAE | 71, 76 | gi\|122106964 | 46592 | 6.28 | 171 | 51 | Magnesium ion binding, phosphopyruvate hydratase activity, glycolysis | Phosphopyruvate hydratase complex | Belongs to the enolase family |
| Succinyl-CoA synthetase beta chain - Aedes *aegypti* | Q16P82_AEDAE | 79 | gi\|122093623 | 48773 | 7.68 | 71 | 12 | ATP binding, ligase activity, metabolic process | | Belongs to the succinate/malate CoA ligase beta subunit family |
| NADP-specific isocitrate dehydrogenase - *Aedes aegypti* | Q16LK8_AEDAE | 88, 89 | gi\|122093450 | 36956 | 8.65 | 169 | 42 | Isocitrate dehydrogenase (NADP+) activity, metabolic process | | Belongs to the isocitrate and isopropylmalate dehydrogenases family |
| Aconitase, mitochondrial - *Aedes aegypti* | Q16KR4_AEDAE | 13 | gi\|122093385 | 85670 | 8.67 | 255 | 35 | Four iron, four sulfur cluster binding, aconitate hydratase activity, iron ion binding, tricarboxylic acid cycle | | |
| Isocitrate dehydrogenase - *Aedes aegypti* | Q17P79_AEDAE | 97 | gi\|122118862 | 43584 | 8.72 | 63 | 20 | Isocitrate dehydrogenase (NAD+) activity, tricarboxylic acid cycle | Mitochondrion | Belongs to the isocitrate and isopropylmalate dehydrogenases family |
| Electron transfer flavoprotein beta-subunit - *Aedes aegypti* | Q17B68_AEDAE | 128 | gi\|122094871 | 22849.0 | 8.98 | 96 | 41 | Electron carrier activity, electron transport, transport | | |
| Thioredoxin reductase - *Aedes aegypti* | Q17GT5_AEDAE | 47 | gi\|122106729 | 53985 | 6.21 | 61 | 14 | Electron carrier activity, FAD binding, NADP binding, oxidoreductase activity, acting on NADH or NADPH, disulfide as acceptor, cell redox homeostasis | Cytoplasm | Belongs to the class-I pyridine nucleotide-disulfide oxidoreductase family. |
| Spermatogenesisassociated factor - *Aedes aegypti* | Q16SH1_AEDAE | 12 | gi[122093853 | 88750 | 5.3 | 147 | 28 | ATP binding, nucleoside-triphosphatase activity | | Belongs to the AAA ATPase family |
| Protein modification machinery | | | | | | | | | | |
| Heat snock cognate 70 - *Aedes aegypti* | Q1HR69_AEDAE | 17 | gi\|122096258 | 72242 | 5.06 | 393 | 47 | Probably plays a role in facilitating the assembly of multimeric protein complexes inside the ER. ATP binding, response to stress | ER lumen | Belongs to theHSP70 family |
| Heat shock cognate 70 *Aedes aegypti* | Q1HQZ5_AEDAE | 25 | gi\|121959204 | 71103 | 5.31 | 237 | 36 | ATP binding, response to stress | | Belongs to theHSP70 family |
| HSP - Aedes *aegypti* | Q16PB5_AEDAE | 16 | gi\|122116881 | 81419 | 4.94 | 147 | 31 | ATP binding, unfolded protein binding, protein foldina, response to stress | | |
| Protein disulfide isomerase - *Aedes aegypti* | Q17L92_AEDAE | 49, 50 | gi\|122107007 | 55211 | 5.5 | 195 | 35 | Isomerase activity, cell redox homeostasis | ER | Belongs to the protein disulfide isomerase family, J51. Contains two thioredoxin domains. |
| ER protein disulfide isomerase - *Aedes aegypti* | Q1HR78_AEDAE | 48,57,58, 59 | gi\|122127522 | 55918 | 4.95 | 241 | 46 | Isomerase activity, cell redox homeostasis | ER | Belongs to the protein disulfide isomerase family. Contains two thioredoxin domains. |
| Calreticulin - *Aedes aegypti* | Q17MI1_AEDAE | 46, 91 | gi\|122107082 | 46698 | 4.42 | 96 | 20 | Calcium ion binding, unfolded protein binding, protein folding | ER | |
| Peptidyl-prolyl *cis-trans* isomerase - Aedes | Q1HRP6_AEDAE | 133 | gi\|122127536 | 21435.0 | 8.71 | 133 | 59 | PPlases accelerate the folding of proteins. It | | Belongs to the cyclophilin-type |
| *aegypti* | | | | | | | | catalyzes the cis-trans isomerization of proline imidic peptide bonds in oligopeptides. peptidyl-prolyl cis-trans isomerase activity, protein folding | | PPlase family. Contains one PPlase cyclophilin-type domain. |
| Endoplasmin - Aedes *aegypti* | Q16KZ2_AEDAE | 11 | gi\|122093400 | 91072 | 4.81 | 269 | 36 | ATP binding, unfolded protein binding, protein folding | | |
| Dihydrolipoyl dehydrogenase - Aedes *aegypti* | Q174D6_AEDAE | 45 | gi\|122106034 | 53626 | 6.36 | 88 | 22 | Dihydrolipoyl dehydrogenase activity, FAD binding, cell redox homeostasis | Cytoplasm | Belongs to the class-I pyridine nucleotide-disulfide oxidoreductase family |

| **Cytoskeletal proteins** | | | | | | | | | | |
|---|---|---|---|---|---|---|---|---|---|---|
| Actin - *Aedes aegypti* | Q178A9_AEDAE | 86 | gi\|122068491 | 41615.7 | 5.29 | 78 | 23 | Actins are highly conserved proteins that are involved in various types of cell motility and are ubiquitously expressed in all eukaryotic cells. ATP binding, protein binding, structural molecule activity | Cytoplasm, cytoskeleton | Belongs to the actin family. |
| Actin 1 - *Aedes aegypti* | ACT1_AEDAE | 86 | gi\|193806368 | 41764.0 | 5.74 | 57 | 21 | Actins are highly conserved proteins that are involved in various types of cell motility and are ubiquitously expressed in all eukaryotic cells | Cytoplasm, cytoskeleton | Belongs to the actin family. |
| Actin 5 *Aedes aegypti* | Q4PKE5_AEDAE | 86 | gi\|75029718 | 41795 | 5.3 | 230 | 48 | Actins are highly conserved proteins that are involved in various types of cell motility and are ubiquitously expressed in all eukaryotic cells. ATP binding, protein binding, structural molecule activity | Cytoplasm, cytoskeleton | Belongs to the actin family |
| Alpha tubulin -*Aedes aegypti* | Q1HR53_AEDAE | 41,42 | gi\|121959249 | 49876 | 5.01 | 155 | 35 | Tubulin is the major constituent of microtubules. It binds 2 mol of GTP, one at an exchangeable site on the beta chain and one at a nonexchangeable site on the alpha-chain. GTP binding, GTPase activity, structural molecule activity, microtubule-based movement, protein polymerization | Microtubule, protein complex | Belongs to the tubulin family. |
| Beta-1 tubulin - *Aedes aegypti* | Q0PHP0_AEDAE | 48 | gi\|122103701 | 48053 | 4.67 | 121 | 20 | Tubulin is the major constituent of microtubules. It binds 2 mol of GTP, one at an exchangeable site on the beta chain and one at a nonexchangeable site on the alpha-chain. GTP binding, GTPase activity, structural molecule activity, microtubule-based movement, protein polymerization | Microtubule, protein complex | Belongs to the tubulin family |
| Myosin heavy chain, nonmuscle or smooth muscle - *Aedes aegypti* | Q178Y4_AEDAE | 1 | gi\|122117926 | 224072 | 5.74 | 140 | 9 | Actin binding, ATP binding, motor activity, striated muscle contraction | Striated muscle thick filament | Contains one myosin head-like domain. |
| Paramyosin, long form-*Aedes aegypti* | Q16RF4_AEDAE | 10 | gi\|122093778 | 103164 | 5.48 | 169 | 20 | | | |
| Moesin/ezrin/radixin-Aedes *aegypti* | Q170J7_AEDAE | 18, 19 | gi\|122068139 | 69024 | 5.75 | 120 | 25 | Cytoskeletal protein binding | Cytoplasm, cytoskeleton, extrinsic to membrane | Contains one FERM domain |
| Gelsolin - *Aedes aegypti* | Q177K9 AEDAE | 82, 83, 84 | gi\|122094688 | 43021 | 9.05 | 328 | 58 | Actin binding | | |

| **Aedes genus specific** | | | | | | | | | | |
|---|---|---|---|---|---|---|---|---|---|---|
| **34 kDa** | | | | | | | | | | |
| Putative 34 kDa family secreted salivary - *Aedes aegypti* | Q1HRW0_AEDAE | 106, 112, 113 | gi\|121959452 | 35698 | 6.1 | 173 | 48 | | | |
| Putative uncharacterized protein - *Aedes aegypti* | Q17F11_AEDAE | 111 | gi\|121956903 | 36170 | 5.2 | 139 | 42 | | | |
| **62 kDa family** | | | | | | | | | | |
| Putative secreted protein - *Aedes aegypti* | Q8T9U8_AEDAE | 28 | gi\|74843993 | 64367 | 5.99 | 199 | 36 | | | |
| Putative uncharacterized protein - *Aedes aegypti* | Q17NC3_AEDAE | 18, 22, 23, 24 | gi\|121957667 | 66197 | 5.8 | 203 | 40 | | | |
| Putative uncharacterized protein - *Aedes aegypti* | Q17NC2_AEDAE | 27 | gi\|122107140 | 64595 | 5.99 | 109 | 22 | | | |

| **Unknown function and ubiquitous** | | | | | | | | | | |
|---|---|---|---|---|---|---|---|---|---|---|
| **Antigen 5 protein family** | | | | | | | | | | |
| **Allergen, putative -** *Aedes aegypti* | Q0IG82_AEDAE | 126 | gi\|122115068 | 24677 | 9.09 | 40 | 16 | | Extracellular region | |
| Putative secreted protein - Aedes Aedes *aegypti* | Q8T9W0_AEDAE | 126 | gi\|74843996 | 29469.0 | 9.02 | 59 | 27 | | Extracellular region | |

| **Other** | | | | | | | | | | |
|---|---|---|---|---|---|---|---|---|---|---|
| Putative uncharacterized protein - *Aedes aegypti* | Q17GF0_AEDAE | 87 | gi\|122106714 | 38583 | 5.27 | 182 | 32 | | | |
| Putative uncharacterized protein - *Aedes aegypti* | Q175J4_AEDAE | 124 | gi\|121956138 | 21245 | 9.04 | 37 | 21 | | | |
| Putative uncharacterized protein - *Aedes aegypti* | Q16QU2_AEDAE | 116 | gi\|122067558 | 30942 | 6.51 | 72 | 23 | | | |
| Putative uncharacterized protein - *Aedes aegypti* | Q16KI2_AEDAE | 8 | gi\|122067251 | 78625 | 5.46 | 57 | 15 | | | |
| Putative uncharacterized protein - *Aedes aegypti* | Q16U69_AEDAE | 21 | gi\|121955332 | 81210 | 9.1 | 90 | 16 | | | |
| Putative uncharacterized protein - *Aedes aegypti* | Q17A21_AEDAE | 8 | gi\|122094812 | 160557 | 5.39 | 44 | 4 | Cell-matrix adhesion | | |
| Putative uncharacterized protein - *Aedes aegypti* | Q16ZT8_AEDAE | 54 | gi\|122105841 | 47542 | 9.13 | 58 | 15 | | | |

| **Unknown function, found in Hematophagous Diptera salivary** | | | | | | | | | | |
|---|---|---|---|---|---|---|---|---|---|---|
| 30 kDa salivary gland allergen Aed a 3 - *Aedes aegypti* | ALL3_AEDAE | 110,118 | gi\|14423642 | 27130.0 | 4.25 | 151 | 30 | | Secreted | |
| Putative 30 kDa allergen-like protein - *Aedes aegypti* | Q8T9T8_AEDAE | 115 | gi\|74819782 | 23569 | 4.57 | 81 | 28 | | | |
| Putative 19.6 kDa secreted protein (precursor) *Aedes aegypti* | Q58HB7_AEDAE | 115, 120, 127 | gi\|74835438 | 21777.0 | 4.78 | 90 | 23 | | | |

### Bibliographic References

1 : Lefevre, T., Thomas, F., Schwartz, A., Levashina, E. et al., Malaria Plasmodium agent induces alteration in the head proteome of their Anopheles mosquito host. Proteomics 2007, 11, 1908-1915.
2 : Wilkins, M.R., Williams, K.L., Cross-species protein identification using amino acid composition, peptide mass fingerprinting, isoelectric point and molecular mass: a theoretical evaluation. J. Theor. Biol. 1997, 186, 7-15.
3 : Missé, D., Cerutti, M., Noraz, N., Jourdan, P., et al., A CD4-independent interaction of human immunodeficiency virus-1 gp120 with CXCR4 induces their cointernalization, cell signaling, and T-cell chemotaxis. Blood 1999, 93, 2454-2462.
4 : Ribeiro, J. M., Arcà, B., Lombardo, F., Calvo, E. et al., An annotated catalogue of salivary gland transcripts in the adult female mosquito, Aedes aegypti. BMC Genomics 2007, 8, 6.
5 : Thisyakorn, U., Nimmannitya, S., Nutritional status of children with dengue hemorrhagic fever. Clin. Infect. Dis. 1993, 16, 295-297.

## Claims

1. Use of at least one of peptides chosen from the peptides of sequence SEQ ID NO 1-15 as *in vitro* epidemiological indicator of exposure to dengue.

2. Use according to claim 1, wherein said peptide is the peptide of sequence SEQ ID NO: 10.

## Patentansprüche

1. Verwendung als *in vitro* epidemiologischen Indikator einer Denguebelastung von wenigsten einem der Peptide der Sequenz SEQ ID NO 1-15.

2. Verwendung nach Anspruch 1, wobei dieses Peptid das Peptid der Sequenz SEQ ID NO: 10 ist.

## Revendications

1. Utilisation d'au moins un des peptides choisis parmi les peptides de séquences SEQ ID NO 1-15 comme indicateur épidémiologique *in vitro* de l'exposition à la dengue.

2. Utilisation selon la revendication 1, dans laquelle ledit peptide est le peptide de séquence SEQ ID NO: 10.


### SEQUENCE LISTING

<110> Institut de la Recherche pour le Développement (IRD)
<120> Proteins/peptides having immunogenic properties and their biological applications
<130> CP 63096-3429
<160> 26
<170> PatentIn version 3.3
<210> 1
   <211> 651
   <212> PRT
   <213> Aedes aegypti
<400> 1
   Met Ala Lys Ala Pro Ala Val Gly Ile Asp Leu Gly Thr Thr Tyr Ser
   1 5 10 15
   Cys Val Gly Val Phe Gln His Gly Lys Val Glu Ile Ile Ala Asn Asp
   20 25 30
   Gln Gly Asn Arg Thr Thr Pro Ser Tyr Val Ala Phe Thr Asp Thr Glu
   35 40 45
   Arg Leu Ile Gly Asp Ala Ala Lys Asn Gln Val Ala Met Asn Pro Thr
   50 55 60
   Asn Thr Ile Phe Asp Ala Lys Arg Leu Ile Gly Arg Lys Phe Asp Asp
   65 70 75 80
   Pro Ala Ile Gln Ala Asp Met Lys His Trp Pro Phe Asp Val Ile Ser
   85 90 95
   Val Glu Gly Lys Pro Lys Ile Gln Val Glu Tyr Lys Gly Glu Thr Lys
   100 105 110
   Asn Phe Phe Pro Glu Glu Ile Ser Ser Met Val Leu Thr Lys Met Lys
   115 120 125
   Glu Thr Ala Glu Ala Tyr Leu Gly Lys Thr Val Ser Asn Ala Val Val
   130 135 140
   Thr Val Pro Ala Tyr Phe Asn Asp Ser Gln Arg Gln Ala Thr Lys Asp
   145 150 155 160
   Ala Gly Thr Ile Ser Gly Leu Asn Val Leu Arg Ile Ile Asn Glu Pro
   165 170 175
   Thr Ala Ala Ala Ile Ala Tyr Gly Leu Asp Lys Lys Thr Ala Gly Glu
   180 185 190
   Arg Asn Val Leu Ile Phe Asp Leu Gly Gly Gly Thr Phe Asp Val Ser
           195                 200                 205
   Ile Leu Ser Ile Asp Asp Gly Ile Phe Glu Val Lys Ser Thr Ala Gly
       210                 215                 220
   Asp Thr His Leu Gly Gly Glu Asp Phe Asp Asn Arg Leu Val Asn His
   225                 230                 235                 240
   Phe Ala Gln Glu Phe Lys Arg Lys His Lys Lys Asp Leu Ser Thr Asn
                   245                 250                 255
   Lys Arg Ala Leu Arg Arg Leu Arg Thr Ala Cys Glu Arg Ala Lys Arg
               260                 265                 270
   Thr Leu Ser Ser Ser Thr Gln Ala Ser Ile Glu Ile Asp Ser Leu Phe
           275                 280                 285
   Glu Gly Thr Asp Phe Tyr Thr Ser Ile Thr Arg Ala Arg Phe Glu Glu
       290                 295                 300
   Leu Asn Ala Asp Leu Phe Arg Ser Thr Met Glu Pro Val Glu Lys Ala
   305                 310                 315                 320
   Ile Arg Asp Ala Lys Met Asp Lys Ala Ser Ile His Asp Ile Val Leu
                   325                 330                 335
   Val Gly Gly Ser Thr Arg Ile Pro Lys Val Gln Lys Leu Leu Gln Asp
               340                 345                 350
   Phe Phe Asn Gly Lys Glu Leu Asn Lys Ser Ile Asn Pro Asp Glu Ala
           355                 360                 365
   Val Ala Tyr Gly Ala Ala Val Gln Ala Ala Ile Leu His Gly Asp Lys
       370                 375                 380
   Ser Glu Glu Val Gln Asp Leu Leu Leu Leu Asp Val Thr Pro Leu Ser
   385                 390                 395                 400
   Leu Gly Ile Glu Thr Ala Gly Gly Val Met Ser Val Leu Ile Lys Arg
                   405                 410                 415
   Asn Thr Thr Ile Pro Thr Lys Gln Thr Gln Thr Phe Thr Thr Tyr Ser
               420                 425                 430
   Asp Asn Gln Pro Gly Val Leu Ile Gln Val Phe Glu Gly Glu Arg Ala
           435                 440                 445
   Met Thr Lys Asp Asn Asn Leu Leu Gly Lys Phe Glu Leu Ser Gly Ile
       450                 455                 460
   Pro Pro Ala Pro Arg Gly Val Pro Gln Ile Glu Val Thr Phe Asp Ile
   465                 470                 475                 480
   Asp Ala Asn Gly Ile Leu Asn Val Thr Ala Leu Glu Lys Ser Thr Asn
                   485                 490                 495
   Lys Glu Asn Lys Ile Thr Ile Thr Asn Asp Lys Gly Arg Leu Ser Lys
               500                 505                 510
   Glu Asp Ile Glu Arg Met Val Asn Glu Ala Glu Lys Tyr Arg Ser Glu
           515                 520                 525
   Asp Glu Lys Gln Lys Glu Thr Ile Ser Ala Lys Asn Ala Leu Glu Ser
       530                 535                 540
   Tyr Cys Phe Asn Met Lys Ala Thr Met Glu Asp Asp Lys Leu Lys Asp
   545                 550                 555                 560
   Lys Ile Thr Asp Ser Asp Lys Thr Leu Ile Met Asp Lys Cys Asn Asp
                   565                 570                 575
   Thr Ile Lys Trp Leu Asp Ala Asn Gln Leu Ala Glu Lys Glu Glu Tyr
               580                 585                 590
   Glu His Arg Gln Lys Glu Leu Glu Ser Val Cys Asn Pro Ile Ile Thr
           595                 600                 605
   Lys Leu Tyr Gln Ser Ala Gly Gly Ala Pro Gly Gly Met Pro Gly Phe
       610                 615                 620
   Pro Gly Gly Ala Pro Gly Ala Gly Ala Gly Ala Ala Pro Gly Ala Gly
   625                 630                 635                 640
   Ser Gly Ser Gly Pro Thr Ile Glu Glu Val Asp
                   645                 650
<210> 2
   <211> 496
   <212> PRT
   <213> Aedes aegypti
<400> 2
   Met Ser Val Asn Arg Thr Ile Ser Ala His Gln Ala Ala Lys Glu His
   1               5                   10                  15
   Val Leu Ala Val Ser Arg Asp Phe Ile Ser Gln Pro Arg Leu Thr Tyr
               20                  25                  30
   Lys Thr Val Ser Gly Val Asn Gly Pro Leu Val Ile Leu Asp Glu Val
           35                  40                  45
   Lys Phe Pro Lys Phe Ala Glu Ile Val Gln Leu Arg Leu Asn Asp Gly
   50 55 60
   Thr Val Arg Ser Gly Gln Val Leu Glu Val Ser Gly Ser Lys Ala Val
   65 70 75 80
   Val Gln Val Phe Glu Gly Thr Ser Gly Ile Asp Ala Lys Asn Thr Val
   85 90 95
   Cys Glu Phe Thr Gly Asp Ile Leu Arg Thr Pro Val Ser Glu Asp Met
   100 105 110
   Leu Gly Arg Val Phe Asn Gly Ser Gly Lys Pro Ile Asp Lys Gly Pro
   115 120 125
   Pro Ile Leu Ala Glu Asp Phe Leu Asp Ile Gln Gly Gln Pro Ile Asn
   130 135 140
   Pro Trp Ser Arg Ile Tyr Pro Glu Glu Met Ile Gln Thr Gly Ile Ser
   145 150 155 160
   Ala Ile Asp Val Met Asn Ser Ile Ala Arg Gly Gln Lys Ile Pro Ile
   165 170 175
   Phe Ser Ala Ala Gly Leu Pro His Asn Glu Ile Ala Ala Gln Ile Cys
   180 185 190
   Arg Gln Ala Gly Leu Val Lys His Thr Gly Lys Ser Val Leu Asp Glu
   195 200 205
   His Glu Asp Asn Phe Ala Ile Val Phe Ala Ala Met Gly Val Asn Met
   210 215 220
   Glu Thr Ala Arg Phe Phe Lys Gln Asp Phe Glu Glu Asn Gly Ser Met
   225 230 235 240
   Glu Asn Val Cys Leu Phe Leu Asn Leu Ala Asn Asp Pro Thr Ile Glu
   245 250 255
   Arg Ile Ile Thr Pro Arg Leu Ala Leu Thr Ala Ala Glu Phe Leu Ala
   260 265 270
   Tyr Gln Cys Glu Lys His Val Leu Val Ile Leu Thr Asp Met Ser Ser
   275 280 285
   Tyr Ala Glu Ala Leu Arg Glu Val Ser Ala Ala Arg Glu Glu Val Pro
   290 295 300
   Gly Arg Arg Gly Phe Pro Gly Tyr Met Tyr Thr Asp Leu Ala Thr Ile
   305 310 315 320
   Tyr Glu Arg Ala Gly Arg Val Glu Gly Arg Asn Gly Ser Ile Thr Gln
   325 330 335
   Ile Pro Ile Leu Thr Met Pro Asn Asp Asp Ile Thr His Pro Ile Pro
   340 345 350
   Asp Leu Thr Gly Tyr Ile Thr Glu Gly Gln Ile Tyr Val Asp Arg Gln
   355 360 365
   Leu His Asn Arg Gln Ile Tyr Pro Pro Val Asn Val Leu Pro Ser Leu
   370 375 380
   Ser Arg Leu Met Lys Ser Ala Ile Gly Glu Gly Met Thr Arg Lys Asp
   385 390 395 400
   His Ser Asp Val Ser Asn Gln Leu Tyr Ala Cys Tyr Ala Ile Gly Lys
   405 410 415
   Asp Val Gln Ala Met Lys Ala Val Val Gly Glu Glu Ala Leu Thr Pro
   420 425 430
   Asp Asp Leu Leu Tyr Leu Glu Phe Leu Thr Lys Phe Glu Lys Asn Phe
   435 440 445
   Ile Ser Gln Gly Asn Tyr Glu Asn Arg Thr Val Phe Glu Ser Leu Asp
   450 455 460
   Ile Gly Trp Gln Leu Leu Arg Ile Phe Pro Lys Glu Met Leu Lys Arg
   465 470 475 480
   Ile Pro Ala Ser Ile Leu Ala Glu Phe Tyr Pro Arg Asp Ser Arg His
   485 490 495
<210> 3
   <211> 530
   <212> PRT
   <213> Aedes aegypti
<400> 3
   Met Pro Ala Asn Ile Ile Met Lys Ile Leu Ile Thr Ser Ile Leu Ile
   1               5                   10                  15
   Leu Lys Leu Ala Ile His Val Val Pro Gln His Leu Ile Ser Ser Gly
               20                  25                  30
   Ala Ser Ala Val Glu Ser Lys Pro Val Ser Ala Arg Pro Thr Tyr Glu
           35                  40                  45
   Asp Tyr Lys Arg Gln Arg Glu Asn Phe Leu Gln Ala Glu Glu Tyr His
       50                  55                  60
   Phe Leu Gly Ala Asn Val Thr Leu Asn Glu Asn Glu Gln Leu Val Asn
   65                  70                  75                  80
   Lys Phe Leu Met Arg Leu Lys Leu Glu Glu Met Val Lys Gly Phe Asn
                   85                  90                  95
   Asp Ser Tyr Asn Phe Ile Pro Ala Arg His Ile Phe Glu Val Leu Asp
               100                 105                 110
   Arg Phe Gly Gln Ser Lys Val Phe Lys Val Ile Gln Arg Leu Pro Lys
           115                 120                 125
   Gly Gly Val Leu His Ala His Asp Met Ala Leu Gly Ser Thr Asp Leu
       130                 135                 140
   Ile Val Asn Ala Thr Tyr Arg Glu Asn Leu Trp Gln Lys Gly Asn Phe
   145                 150                 155                 160
   Gly Val Ser His Gly Pro Gln Phe Lys Phe Ser Lys Glu Lys Pro Gly
                   165                 170                 175
   Lys Glu Trp Ser Leu Val Ser Glu Ile Arg Gln Trp Met Thr Asp Lys
               180                 185                 190
   Val Tyr Asp Ala Lys Val Gly Glu Ile Phe Ser Leu Tyr Asn Ala Asp
           195                 200                 205
   Pro Leu Asn Ala Tyr Lys Ser Leu Asp Asp Val Trp Ser Lys Phe Gln
       210                 215                 220
   Asn Leu Phe Gly Ser Leu Ala Pro Leu Ile Thr Phe Ala Pro Val Trp
   225                 230                 235                 240
   Arg Gln Tyr Tyr His Asp Ser Leu Lys Gln Phe Tyr Asp Asp His Val
                   245                 250                 255
   Gln Tyr Leu Glu Phe Arg Gly Val Leu Pro Asp Val Tyr Asp Leu Asp
               260                 265                 270
   Gly Lys Ile Tyr Ser Ala Glu Glu Ile Val Gln Met Tyr Tyr Glu Glu
           275                 280                 285
   Thr Glu Glu Phe Lys Ser Ser His Pro Glu Phe Ile Gly Ala Lys Phe
       290                 295                 300
   Ile Tyr Ala Pro Gly Arg Phe Ala Thr Asp Asp Glu Phe Leu Lys Ile
   305                 310                 315                 320
   Ile Asp Thr Ala Lys Arg Leu His Lys Lys Phe Pro Thr Phe Leu Ala
                   325                 330                 335
   Gly Phe Asp Leu Val Gly Gln Glu Asp Pro Gly Arg Ser Leu Leu Glu
               340                 345                 350
   Phe Ala Pro Ala Leu Leu Lys Leu Pro Ala Ser Ile Asn Phe Phe Phe
   355 360 365
   His Ala Gly Glu Thr Asn Trp Tyr Gly Met Lys Thr Asp Gln Asn Leu
   370 375 380
   Ile Asp Ala Val Leu Leu Gly Ser Lys Arg Ile Gly His Gly Phe Ala
   385 390 395 400
   Val Leu Lys His Pro Lys Val Leu Lys Glu Ile Lys Arg Arg Gln Ile
   405 410 415
   Cys Ile Glu Ile Asn Pro Ile Ser Asn Gln Val Leu Lys Leu Val Gln
   420 425 430
   Asp Gln Arg Asn His Pro Ala Ala Leu Leu Phe Ser Asp Asn Tyr Pro
   435 440 445
   Val Val Val Ser Ser Asp Asp Pro Ser Phe Trp Arg Ser Thr Pro Leu
   450 455 460
   Ser His Asp Phe Tyr Val Ala Phe Thr Gly Ile Ala Ser Ala Lys Gln
   465 470 475 480
   Asp Leu Arg Leu Leu Lys Gln Leu Ala Leu Asn Ser Ile Glu Tyr Ser
   485 490 495
   Ala Met Asn Ser Glu Glu Lys Thr Ser Ala Lys Glu Lys Trp Ser Gln
   500 505 510
   Ala Trp His Asp Gln Ile Ser Ala Leu Ala Thr Asp Ile Val Ala Gly
   515 520 525
   Ser Val
   530
<210> 4
   <211> 562
   <212> PRT
   <213> Aedes aegypti
<400> 4
   Met Ala Gly Arg Pro Gly Tyr Ser Glu Val Ile Phe Leu Tyr Val Val
   1 5 10 15
   Ser Val Ala Val Ile Ala Arg Ala Thr Asp Asn Met Pro Val Asn Lys
   20 25 30
   Asp Val Ser Lys Leu Phe Pro Leu Thr Leu Ile His Ile Asn Asp Leu
   35 40 45
   His Ala Arg Phe Glu Glu Thr Asn Met Lys Ser Asn Val Cys Thr Gln
   50 55 60
   Lys Asp Gln Cys Ile Ala Gly Ile Ala Arg Val Tyr Gln Lys Ile Lys
   65 70 75 80
   Asp Leu Leu Lys Glu Tyr Glu Ser Lys Asn Pro Ile Tyr Leu Asn Ala
   85 90 95
   Gly Asp Asn Phe Gln Gly Thr Leu Trp Tyr Asn Leu Leu Arg Trp Asn
   100 105 110
   Val Thr Ala Asp Phe Ile Lys Lys Leu Lys Pro Ala Ala Met Thr Leu
   115 120 125
   Gly Asn His Glu Phe Asp His Thr Pro Lys Gly Leu Ala Pro Tyr Leu
   130 135 140
   Ala Glu Leu Asn Lys Glu Gly Ile Pro Thr Ile Val Ala Asn Leu Val
   145 150 155 160
   Met Asn Asn Asp Pro Asp Leu Lys Ser Ser Lys Ile Pro Lys Ser Ile
   165 170 175
   Lys Leu Thr Val Gly Lys Arg Lys Ile Gly Ile Ile Gly Val Leu Tyr
   180 185 190
   Asp Lys Thr His Glu Ile Ala Gln Thr Gly Lys Val Thr Leu Ser Asn
   195 200 205
   Ala Val Glu Ala Val Arg Arg Glu Ala Ala Ala Leu Lys Lys Asp Asn
   210 215 220
   Ile Asp Ile Ile Val Val Leu Ser His Cys Ser Tyr Glu Glu Asp Lys
   225 230 235 240
   Lys Ile Ala Ala Glu Ala Gly Asp Asp Ile Asp Val Ile Val Gly Ala
   245 250 255
   His Ser His Ser Phe Leu Tyr Ser Pro Asp Ser Lys Gln Pro His Asp
   260 265 270
   Pro Lys Asp Lys Val Glu Gly Pro Tyr Pro Thr Leu Val Glu Ser Lys
   275 280 285
   Asn Lys Arg Lys Ile Pro Ile Val Gln Ala Lys Ser Phe Gly Lys Tyr
   290 295 300
   Val Gly Arg Leu Thr Leu Tyr Phe Asp Glu Glu Gly Glu Val Lys Asn
   305 310 315 320
   Trp Glu Gly Tyr Pro Val Phe Ile Asp His Lys Val Gln Gln Asp Pro
                   325                 330                 335
   Gln Ile Leu Lys Asp Leu Val Pro Trp Arg Ala Lys Val Glu Ala Ile
               340                 345                 350
   Gly Ser Thr Val Val Gly Glu Thr Met Ile Glu Leu Asp Arg Asp Ser
           355                 360                 365
   Cys Arg Asp Gln Glu Cys Thr Leu Gly Val Leu Tyr Ala Asp Gly Phe
       370                 375                 380
   Ala Asp Gln Tyr Thr Asn Asp Thr Phe Arg Pro Phe Ala Ile Ile Gln
   385                 390                 395                 400
   Ala Gly Asn Phe Arg Asn Pro Ile Lys Val Gly Lys Ile Thr Asn Gly
                   405                 410                 415
   Asp Ile Ile Glu Ala Ala Pro Phe Gly Ser Thr Ala Asp Leu Ile Arg
               420                 425                 430
   Leu Lys Gly Ala Asp Ile Trp Asp Val Ala Glu His Ser Phe Ala Leu
           435                 440                 445
   Asp Asp Glu Gly Arg Thr Asn Cys Leu Gln Val Ser Gly Leu Arg Ile
       450                 455                 460
   Val Ile Asp Ile Ser Lys Pro Val Arg Ser Arg Val Lys Lys Ile Glu
   465                 470                 475                 480
   Val Met Asp Tyr Thr Asn Pro Lys Ser Asp Lys Leu Lys Pro Leu Asp
                   485                 490                 495
   Lys Glu Ala Glu Tyr Tyr Ile Val Val Pro Ser Tyr Leu Ala Asp Gly
               500                 505                 510
   Lys Asp Gly Phe Ser Ala Met Lys Arg Ala Thr Ala Arg Arg Thr Gly
           515                 520                 525
   Pro Leu Asp Ser Asp Val Phe Lys Asn Tyr Val Glu Lys Ile Lys Lys
       530                 535                 540
   Val Asp Asn Leu Lys Leu Gly Arg Val Ile Val Cys Lys Gly Ser Lys
   545                 550                 555                 560
   Cys Thr
<210> 5
   <211> 572
   <212> PRT
   <213> Aedes aegypti
<400> 5
   Met Ile Asp Gln Cys Ala Cys Ser His Gln Leu Ser Ala Ala Leu Ser
   1               5                   10                  15
   Thr Glu Asp Met Leu Arg Thr Ser Ser Ile Val Phe Leu Thr Cys Cys
               20                  25                  30
   Leu Thr Phe Leu Ile Glu Gly Ser Ser Phe Lys Leu Lys Ile Ile His
           35                  40                  45
   Phe Asn Asp Ile His Ala Arg Phe Asp Glu Val Thr Asn Ser Ser Ser
       50                  55                  60
   Pro Cys Ser Gly Asn Gly Glu Thr Cys Val Ala Gly Ile Ala Arg Leu
   65                  70                  75                  80
   Val Thr Thr Ile Glu Lys Leu Arg Lys Gln Asn Glu Asn His Leu Val
                   85                  90                  95
   Leu Asn Ala Gly Asp Val Phe Gln Gly Thr Ile Trp Tyr Thr Leu Leu
               100                 105                 110
   Lys Trp Asn Val Ser Gln Gln Phe Met Asn Met Val Lys Ala Asp Ala
           115                 120                 125
   Met Thr Leu Gly Asn His Glu Phe Asp Asp Ser Phe Pro Val Leu Ile
       130                 135                 140
   Pro Phe Leu Glu Asn Thr Lys Asn Val Thr Pro Val Val Val Ser Asn
   145                 150                 155                 160
   Leu Val Phe Pro Lys Gln Leu Ser Arg Asp Val Thr Lys Phe Arg Ser
                   165                 170                 175
   Leu Ile Lys Glu Asp Pro Leu Val Leu Thr Val Gly Gly Gln Ser Ile
               180                 185                 190
   Gly Ile Ile Gly Val Ile Phe Asp Glu Thr Asp Lys Ile Gly Asn Ser
           195                 200                 205
   Asp Pro Leu Lys Phe Lys Ser Ser Ile Glu Thr Val Arg Ile Ala Ala
       210                 215                 220
   Lys Gln Leu Lys Ser Lys Gly Val Asn Ile Ile Ile Val Leu Ser His
   225                 230                 235                 240
   Cys Gly Val Phe Asp Asp Lys Lys Ile Ala Glu Gln Ala Gly Glu Asp
                   245                 250                 255
   Ile Asp Ile Ile Val Gly Gly His Thr His Thr Leu Leu Tyr Asn Gly
   260 265 270
   Asp Pro Pro Ser Lys His Ala Ala Leu Asp Lys Tyr Pro Ile Val Val
   275 280 285
   Glu Thr Gly Asn Asn His Lys Val Leu Ile Val Gln Ala Phe Cys His
   290 295 300
   Gly His Tyr Val Gly Asn Ile Asp Leu Thr Phe Asp Asp Glu Gly Glu
   305 310 315 320
   Ile Thr Ala Phe Glu Gly Gln Pro Ile Tyr Gln Glu Asn Arg Ile Glu
   325 330 335
   Lys Asn Ala Leu Val Glu Ala Arg Val Arg Glu Leu Arg Lys Asp Val
   340 345 350
   Glu Val Lys Ser Leu Val Lys Val Gly Glu Ser Lys Leu Glu Leu Ser
   355 360 365
   Asn Asp Cys Arg Leu Lys Asp Cys Thr Phe Gly Ser Val Leu Ala Asp
   370 375 380
   Ala Tyr Val Trp His Phe Arg Ser Arg Ser Asn Ala Pro Met Ile Ala
   385 390 395 400
   Met Ile His Pro Gly Asn Phe Arg Ile Ser Leu Ala Ala Gly Ala Ile
   405 410 415
   Thr Arg Gly Gln Ile Leu Thr Ala Leu Pro Phe Asn Ser Asn Ala Asn
   420 425 430
   Arg Val Thr Val Leu Gly Ser Thr Ile Lys Lys Ala Ile Glu Phe Gly
   435 440 445
   Thr Ser Ile Asn Pro Arg Arg Cys Ser Phe Asn Ala Leu Gln Thr Ala
   450 455 460
   Gly Ile Lys Ile Asp Val Asp Tyr Gly Lys Pro Val Gly Asn Arg Thr
   465 470 475 480
   Val Ile Leu Leu Lys Thr Gly Gly Lys Tyr Lys Arg Leu Val Glu Ser
   485 490 495
   Lys Lys Tyr Asp Ile Leu Val Asn Ser Tyr Val Phe Lys Gly Gly Asp
   500 505 510
   Gly Phe Asp Met Phe Lys His Leu Ala Val Lys Gly Arg Ala Pro Phe
   515 520 525
   Asp Ala Glu Leu Leu Glu Gln Tyr Ile Val Ala Arg Lys Gly Ile Gln
       530                 535                 540
   Lys Ser Gly Leu Leu Gln Ser Arg Met Asn Val Ser His Val Glu Lys
   545                 550                 555                 560
   Ala Leu Ser Glu Val Lys Ser Cys Lys Gln Ser Arg
                   565                 570
<210> 6
   <211> 418
   <212> PRT
   <213> Aedes aegypti
<400> 6
   Met Cys Ser Thr Gly Phe Cys Leu Val Phe Phe Leu Ala Gln Val Val
   1               5                   10                  15
   Phe Gln Met Asn Tyr Ser Glu Gln Gln Thr Thr Val Val Met Glu Asn
               20                  25                  30
   Gly Ala Ile Ser Glu Ser Glu Ile Asn Val Asp Ile Val Met Glu Gln
           35                  40                  45
   Tyr Ile Leu Lys Phe Tyr Thr Lys Arg Phe Val Glu Gly Gln Asn Leu
       50                  55                  60
   Val Val Ala Pro Leu Leu Thr Phe Arg Val Phe Met Ser Leu Tyr Lys
   65                  70                  75                  80
   Ala Met Asp Ala Ser Ala Lys Phe Asp Leu His Ser Leu Leu Gly Ile
                   85                  90                  95
   Gln Gln Asp Thr Ser Val Glu Lys Met Ser Glu Ile Glu Ala Phe Ala
               100                 105                 110
   Asn Lys His Thr Leu Pro Val Asp Glu Lys Gln Ile Ser Val Glu Thr
           115                 120                 125
   Arg Leu Tyr Tyr Asp Lys Ser Ile Gly Asn Ala Arg Ser Val Leu Thr
       130                 135                 140
   Ala Lys Ser Leu Lys Pro Ile Gly Thr Ser Phe Ser Asp Lys Arg Ala
   145                 150                 155                 160
   Phe Cys Glu Lys Val Asn Ser Trp Ile Arg Asn Ala Pro Ile Lys Gly
                   165                 170                 175
   Thr Asp Asn Leu Val Arg Asp Tyr Asp Leu Asn Asn Glu Thr Gln Ala
               180                 185                 190
   Phe Val Ala Gly Ala Leu Ser Ile Tyr Trp Asn Thr Gln Leu Lys Ser
   195 200 205
   Ser Thr Asp Gln Lys Gly Phe Gln Gly Glu Asn Val Lys Phe Leu Glu
   210 215 220
   Gly Ser Ile Ser Ala Gly Tyr Ala Lys Leu Asp Asn Leu Lys Val Glu
   225 230 235 240
   Val Val Glu Leu Ile Ser Asp Lys Val Asp Gly Val Lys Leu Trp Leu
   245 250 255
   Ile Met Pro Asp Arg Ala Ser Ser Ile Lys Asp Phe Asn Asp Gln Leu
   260 265 270
   Ser Val Glu Ser Ile Arg Gln Ile Glu Asn Gly Leu Thr Ala Gln Lys
   275 280 285
   Val Asp Val Ser Leu Ala Leu Pro Met Val Thr Ile Glu Tyr Asn Ser
   290 295 300
   Gln Glu Asp Ala Tyr Val Thr Glu Val Phe Glu Val Phe Ser Ser Leu
   305 310 315 320
   Phe Thr Lys Pro Ser Val Lys Leu Val Asp Gly Lys Asp Asp Leu Tyr
   325 330 335
   Val Ile Lys Asn Phe Leu Met Lys Cys Ile Leu Arg Phe Val Glu Ser
   340 345 350
   Asp Ala Ser Ala Asp Ser Lys Ala Gln Ser Thr Gly Met Leu Val Lys
   355 360 365
   Phe Asp Arg Pro Phe Val Met Met Met Leu Ser Lys Glu Gly Asn Val
   370 375 380
   Pro Ile Leu Leu Ala Asn Tyr Phe Ser Pro Thr Asp Lys Leu Arg Ala
   385 390 395 400
   Leu Glu Ala Lys Glu Arg Arg Leu Lys Ala Glu Ala Asn Glu His Leu
   405 410 415
   Asp Leu
<210> 7
   <211> 417
   <212> PRT
   <213> Aedes aegypti
<400> 7
   Met Asn Leu Trp Ile Ile Gly Phe Cys Ser Ile Tyr Phe Ala Cys Ser
   1               5                   10                  15
   Val Arg Ser Gln Phe Thr Ser Val Pro Val Ser Tyr Asp Ala Gln Asn
   20 25 30
   Asp His Asn Glu Phe Ser Trp Asn Ala Phe Lys Lys Val Phe Thr Asp
   35 40 45
   Tyr Lys Glu Asn Phe Val Met Ser Pro Tyr Ser Leu Arg Arg Leu Phe
   50 55 60
   Ser Cys Phe Gln Gly Val Lys Leu Leu Thr Ser Ala Ser Gly Thr Asn
   65 70 75 80
   Leu Gln Gln Glu Leu Ser Asn Val Leu Lys Ile Val Pro Asn Gln Gln
   85 90 95
   Pro Ser Gly Gln Asp His Arg Pro Tyr Val Glu Gln Trp Val Arg Tyr
   100 105 110
   Ser Ser Ala Lys Tyr Leu Asn Arg Thr Ala Met Ala Val Ala Ile Gly
   115 120 125
   Ser Glu Lys Val Ser Thr Val Tyr Glu Ser Ile Ile Asn Asn Cys Val
   130 135 140
   Ile Tyr Thr Gly His Leu Gln Pro Ser Asn Ala Gln Arg Met Gly Gln
   145 150 155 160
   Val Ile Asn Asp Ala Leu Lys Asn Ile Thr Asn Asn Ala Val Gln Ser
   165 170 175
   Tyr Leu Thr Asp Thr Asp Ile Asn Pro Asn Trp Lys Phe Phe Ala Ile
   180 185 190
   Asp Ser Trp Gln Phe Glu Gly Leu Trp Lys Phe Lys Phe Gln Glu Glu
   195 200 205
   Phe Ser Ala Thr Cys Tyr Phe Tyr Ala Ser Arg Glu Lys Lys Gly Leu
   210 215 220
   Thr Lys Phe Leu Tyr Leu Glu Glu Met Leu Lys Tyr Gly Asn Phe Pro
   225 230 235 240
   Glu Trp Asn Val Gln Ala Val Glu Leu Pro Tyr His Asp Gln Ser Pro
   245 250 255
   Leu Ser Cys Leu Leu Met Met Pro Leu Asp Gly Asn Tyr Glu Ser Leu
   260 265 270
   Ile His Ser Met Asn Gln Ser Arg Phe Lys Glu Val Leu Ser Lys Leu
   275 280 285
   Asn Glu Ile Lys Thr Thr Val Arg Ile Pro Gln Phe Gly Leu Gln Thr
       290                 295                 300
   Thr Val Pro Gly Arg Gln Leu Leu Glu Ser Met Gly Met Lys Val Pro
   305                 310                 315                 320
   Phe Asn Gln Gly Val Phe Lys Val Phe Glu Gln Gly Gln Asp Val Ala
                   325                 330                 335
   Leu Gly Glu Ile Val Gln Lys Met Glu Met Ser Ile Ala Ala Asp Gly
               340                 345                 350
   Glu Lys Gln Ala Gln Ser Phe Val Asp Lys Arg Gln Asp Lys Gln Phe
           355                 360                 365
   Thr Ala His Gln Pro Phe Leu Phe Val Val Tyr Asp Arg Asn Glu Leu
       370                 375                 380
   Val Pro Ile Leu Val Gly Phe Tyr Leu Lys Thr Pro Pro Glu Ala Ala
   385                 390                 395                 400
   Met Gly Leu Glu Asp Lys Gln Lys Cys Asp Asp Pro Pro Val Gly Tyr
                   405                 410                 415
   Gln
<210> 8
   <211> 291
   <212> PRT
   <213> Aedes aegypti
<400> 8
   Met Asn Arg Gln Leu Trp Ile Ile Ile Phe Ala Ile Leu Cys Val Ala
   1               5                   10                  15
   Gln Ala Glu Glu Asp Asn Pro Thr Thr Glu Lys Met Glu Glu Leu Gly
               20                  25                  30
   Ile Ala Thr Ile Asn Asn Phe Thr Arg Glu Phe Tyr Ser Tyr Val Glu
           35                  40                  45
   Ala Val Ser Gln Val Leu Ala Asp Leu Glu Leu Thr Thr Thr Ala Ser
       50                  55                  60
   Ile Thr Gln Ile Lys His Arg Ile Lys His Leu Leu Gln Glu Lys Cys
   65                  70                  75                  80
   Asn Leu Cys Ser Ala Lys Ala Glu Gly Pro Ala Leu Asp Gln Gly Tyr
                   85                  90                  95
   Val Thr Thr Ser Asn Gly Ser Val Ile Pro Val Ser Tyr Glu Gln Thr
   100 105 110
   Arg Phe Gly Gly Gly Trp Ile Val Leu Met Gln Arg Tyr Asp Gly Thr
   115 120 125
   Val Arg Phe Asn Arg Ser Trp Ala Glu Tyr Arg Asp Gly Phe Gly Met
   130 135 140
   Val Gly His Glu Phe Trp Leu Gly Leu Glu Arg Ile His Gln Met Thr
   145 150 155 160
   Lys Asp Ala Glu Tyr Glu Leu Met Ile Glu Met Gln Asp Phe Glu Gly
   165 170 175
   Asn Tyr Lys Tyr Ala Gly Tyr Asp Ala Phe Ala Val Gly Pro Glu Glu
   180 185 190
   Glu Arg Tyr Pro Leu Ala Lys Val Gly Lys Phe Asn Lys Thr Ala Tyr
   195 200 205
   Val Asp Ser Phe Gly Lys His Arg Gly Tyr Gly Phe Ser Thr Tyr Asp
   210 215 220
   Asn Asp Asp Asn Gly Cys Ser Asn Gln Tyr Gly Arg Gly Gly Trp Trp
   225 230 235 240
   Tyr Tyr Arg Lys Ser Cys Phe Gly Ala Ser Leu Thr Gly Ile Trp Gln
   245 250 255
   Asn Lys Gln Asp Trp Lys Ser Ile Ser Trp Val Trp Phe Ser Thr Glu
   260 265 270
   Lys Lys Gln Val Pro Leu Lys Phe Ala Arg Met Met Met Arg Leu Lys
   275 280 285
   Thr Ala Glu
   290
<210> 9
   <211> 290
   <212> PRT
   <213> Aedes aegypti
<400> 9
   Met Ile Leu Gln Phe Trp Phe Val Thr Phe Ser Val Leu Phe Ala Ala
   1 5 10 15
   Arg Ala Asp Glu Asn His Ser Ile Leu Ile Lys Leu Asn Asp Leu Asp
   20 25 30
   His Arg Phe Thr Gln Met Phe Ser Gln Gln Phe Tyr Arg His Thr Arg
   35 40 45
   Glu Val Thr Asp Arg Val Ser Ala Leu Lys Ala Ser Ile Asp Thr Asn
   50 55 60
   Leu Leu Glu Leu Asp Gln Gln Ile Gln Gln Ala Leu Asp Gly Ile Gln
   65 70 75 80
   Ser Asn Glu Ser Ser Ser Ser Thr Ser Ala Thr Lys Ser Ser Gly Leu
   85 90 95
   Thr Thr Ile Pro Ile Gly Ser Glu Pro Arg Val Pro Ala Leu Tyr Glu
   100 105 110
   Arg Glu Arg Tyr Gly Gly Asp Trp Leu Val Val Met His Arg Tyr Asp
   115 120 125
   Gly Ser Val Lys Phe Asp Arg Thr Trp Ala Glu Tyr Arg Asp Gly Phe
   130 135 140
   Gly Met Val Gly Gln Glu Phe Trp Tyr Gly Leu Glu Arg Leu His Gln
   145 150 155 160
   Leu Thr Lys Glu Lys Ser Tyr Glu Leu Met Val Glu Met Glu Asp Phe
   165 170 175
   Asn Gly Asn Leu Lys Tyr Ala Trp Tyr Asp Lys Phe Val Val Gly Pro
   180 185 190
   Glu Glu Gln Arg Tyr Ala Leu Val Glu Leu Gly Thr Phe Asn Gly Thr
   195 200 205
   Thr Asp Gly Asp Ser Leu Lys Pro His Lys Gly Ser Gly Phe Ser Thr
   210 215 220
   Tyr Asp Asn Asp Asp Phe Gly Cys Ser Asn Lys Tyr Ala Lys Gly Gly
   225 230 235 240
   Trp Trp Tyr Tyr Ser Gly Lys Cys Tyr Gly Ser Ser Leu Thr Gly Ile
   245 250 255
   Trp Lys Asn Glu Leu Ala Tyr Ser Ser Ile Val Trp Val Lys Phe Ser
   260 265 270
   Asp Val Ser Asn Thr Pro Leu Lys Leu Val Arg Met Met Ile Arg Pro
   275 280 285
   Lys Asn
   290
<210> 10
   <211> 312
   <212> PRT
   <213> Aedes aegypti
<400> 10
   Met Ser Pro Ser Asn Lys Ile Leu Val Leu Leu Leu Phe Pro Ile Leu
   1               5                   10                  15
   Leu Val Ser Ser His Pro Ile Pro Ala Glu Asp Pro Ala Lys Gln Cys
               20                  25                  30
   Asn Leu Ser Glu Asp Asp Leu Thr Lys Leu Lys Ala Ala Ile Ser Gly
           35                  40                  45
   Ala Ser Ser Ala Lys Ala Ala Asn Glu Asp Ile Leu Pro Asn Thr Thr
       50                  55                  60
   Leu Ala Ala Cys Pro Met Leu Lys Asn Phe Thr Glu Met Leu Lys Thr
   65                  70                  75                  80
   Val Ala Thr Asp Met Glu Val Leu Lys Thr Gln Gly Val Ser Asn Met
                   85                  90                  95
   Glu Val Gln Leu Leu Arg Glu Ser Phe Glu Glu Lys Leu Asn Asp Leu
               100                 105                 110
   Ala Lys Asn Lys Asp Ile Phe Glu Arg Gln Ala Asn Gln Asp Thr Ser
           115                 120                 125
   Lys Ala Glu Gly Glu Met Val Glu Lys Ile Asn Lys Leu Gln Leu Glu
       130                 135                 140
   Met Ala Lys Leu Gln Glu Glu Ile Glu Glu Gln Thr Lys Gln Met Tyr
   145                 150                 155                 160
   Val Asp Met Ile Glu Tyr Ile Phe Glu Arg Leu Lys Met Asn Asp Thr
                   165                 170                 175
   Glu Ala Ile Asp Ser Tyr Ala Gln Ile Val Met Lys Thr Lys Met His
               180                 185                 190
   Glu Leu Ile Met Lys Leu Lys Thr Asp Arg Leu Val Leu Trp Glu Met
           195                 200                 205
   Val Lys Tyr Val Glu Gly Lys Lys Asn Lys Trp Val Gly Arg Lys Val
       210                 215                 220
   Leu Asn Thr Ile Leu Asp Gln Val Asn Lys Leu Lys Leu Tyr Lys Pro
   225                 230                 235                 240
   Glu Glu Val Glu Ile Gly Lys Asn Ser Leu Val Val Val Trp Cys Trp
   245 250 255
   Lys Phe Asn Ser Glu Thr Val Tyr Gly Thr Thr Asp Glu Asp Gln Lys
   260 265 270
   Ser Phe His Leu Ala Lys Leu Phe Phe Pro Lys Glu Lys Gly Cys Lys
   275 280 285
   Glu Cys Ala Asn Val Lys Ser Arg Thr Met Cys Asn Asn Asp Tyr Pro
   290 295 300
   Lys Val Met Val Lys Ala Phe Gly
   305 310
<210> 11
   <211> 321
   <212> PRT
   <213> Aedes aegypti
<400> 11
   Met Lys Leu Pro Leu Leu Leu Ala Ile Val Thr Thr Phe Ser Val Val
   1               5                   10                  15
   Ala Ser Thr Gly Pro Phe Asp Pro Glu Glu Met Leu Phe Thr Phe Thr
               20                  25                  30
   Arg Cys Met Glu Asp Asn Leu Glu Asp Gly Pro Asn Arg Leu Pro Met
           35                  40                  45
   Leu Ala Lys Trp Lys Glu Trp Ile Asn Glu Pro Val Asp Ser Pro Ala
       50                  55                  60
   Thr Gln Cys Phe Gly Lys Cys Val Leu Val Arg Thr Gly Leu Tyr Asp
   65                  70                  75                  80
   Pro Val Ala Gln Lys Phe Asp Ala Ser Val Ile Gln Glu Gln Phe Lys
                   85                  90                  95
   Ala Tyr Pro Ser Leu Gly Glu Lys Ser Lys Val Glu Ala Tyr Ala Asn
               100                 105                 110
   Ala Val Gln Gln Leu Pro Ser Thr Asn Asn Asp Cys Ala Ala Val Phe
           115                 120                 125
   Lys Ala Tyr Asp Pro Val His Lys Ala His Lys Asp Thr Ser Lys Asn
       130                 135                 140
   Leu Phe His Gly Asn Lys Glu Leu Thr Lys Gly Leu Tyr Glu Lys Leu
   145                 150                 155                 160
   Gly Lys Asp Ile Arg Gln Lys Lys Gln Ser Tyr Phe Glu Phe Cys Glu
                   165                 170                 175
   Asn Lys Tyr Tyr Pro Ala Gly Ser Asp Lys Arg Gln Gln Leu Cys Lys
   180 185 190
   Ile Arg Gln Tyr Thr Val Leu Asp Asp Ala Leu Phe Lys Glu His Thr
   195 200 205
   Asp Cys Val Met Lys Gly Ile Arg Tyr Ile Thr Lys Asn Asn Glu Leu
   210 215 220
   Asp Ala Glu Glu Val Lys Arg Asp Phe Met Gln Val Asn Lys Asp Thr
   225 230 235 240
   Lys Ala Leu Glu Lys Val Leu Asn Asp Cys Lys Ser Lys Glu Pro Ser
   245 250 255
   Asn Ala Gly Glu Lys Ser Trp His Tyr Tyr Lys Cys Leu Val Glu Ser
   260 265 270
   Ser Val Lys Asp Asp Phe Lys Glu Ala Phe Asp Tyr Arg Glu Val Arg
   275 280 285
   Ser Gln Ile Tyr Ala Phe Asn Leu Pro Lys Lys Gln Val Tyr Ser Lys
   290 295 300
   Pro Ala Val Gln Ser Gln Val Met Glu Ile Asp Gly Lys Gln Cys Pro
   305 310 315 320
   Gln
<210> 12
   <211> 332
   <212> PRT
   <213> Aedes aegypti
<400> 12
   Met His Ser Pro Lys Ser Phe Leu Leu Leu Ala Val Val Phe Val Ala
   1 5 10 15
   Leu Arg Val Thr Ala Ala Pro Leu Trp Asn Ala Lys Asn Pro Glu Gln
   20 25 30
   Leu Gln Tyr Ile Ala Ala Arg Cys Met Glu Glu Trp Ser Pro Lys Ala
   35 40 45
   Lys Asp Pro Lys Ala Ala Leu Lys Asn Trp Met Glu Trp Lys Leu Gln
   50 55 60
   Pro Ser Asn Glu Glu Ala Thr Gln Cys Tyr Thr Lys Cys Met Leu Glu
   65 70 75 80
   Asn Ile Gly Tyr Tyr Glu Pro Gly Glu Lys Arg Leu Lys Gly Val Arg
                   85                  90                  95
   Val Met Gln Gln Trp Glu Thr Phe Asn Arg Tyr Gln Ser Ala Asp Arg
               100                 105                 110
   Asn Lys Val His Asp Leu Thr Asp Thr Phe Asp Phe Ile Lys Pro Leu
           115                 120                 125
   Lys Ser Ser Ser Cys Ser Asp Val Phe Asn Ala Tyr Lys Asp Val His
       130                 135                 140
   Ala Lys His Leu Glu Thr Ile Lys Ala Ile Leu Phe Cys Asp Gly Lys
   145                 150                 155                 160
   Ser Ala Glu Lys Tyr Tyr Lys Asp Lys Gly Lys Asn Val Lys Gln Lys
                   165                 170                 175
   Gly Glu Ser Ile Phe Val His Cys Glu Glu Ile His Tyr Pro Val Gly
               180                 185                 190
   Ser Pro Gln Arg Asn Glu Leu Cys Lys Val Arg Lys Tyr Glu Leu Gly
           195                 200                 205
   Thr Gly Lys Pro Phe Glu Asn Leu Met Glu Cys Ile Phe Lys Gly Val
       210                 215                 220
   Arg Tyr Phe Asn Asp Lys Asn Glu Leu Asn Ile Asp Glu Ile Ala Arg
   225                 230                 235                 240
   Asp Phe Thr Gln Val Gly Lys Lys Pro Asp Ala Val Lys Ala Ala Met
                   245                 250                 255
   Glu Asn Cys Lys Ser Lys Thr Lys Glu Thr Asp Pro Gly Lys Lys Ala
               260                 265                 270
   Val Glu Tyr Tyr Lys Cys Leu Leu Ala Asp Ser Lys Val Lys Lys Asp
           275                 280                 285
   Phe Met Glu Ala Phe Asp Tyr Arg Glu Ile Arg Ser Lys Asp Tyr Tyr
       290                 295                 300
   Ala Gln Ile Thr Gly Lys Leu Lys Pro Tyr Ser Ala Ser Asp Val Arg
   305                 310                 315                 320
   Lys Glu Val Asn Asp Ile Asp Ser Asn Lys Cys Val
                   325                 330
<210> 13
   <211> 568
   <212> PRT
   <213> Aedes aegypti
<400> 13
   Met Lys Leu Lys Val Tyr Ile Cys Gln Val Ile Phe Ser Phe Leu Ala
   1               5                   10                  15
   Val Ser Val Phe Cys Glu Glu Asn Cys Asn Ile Pro Glu Ser Glu Leu
               20                  25                  30
   Ser Lys Ile Asp His Val Leu Arg His Met Glu Lys Pro Ile Tyr Ser
           35                  40                  45
   Glu Glu Gln Phe Ala Ser Asp Asn Glu Glu Cys Thr Asn Leu Leu Asn
       50                  55                  60
   Gly Ile His Ala Gln Leu Arg Arg Leu Thr Gln Arg Tyr Lys Leu Met
   65                  70                  75                  80
   Asn Lys Gly Tyr Val Lys Val Glu Glu Tyr Gln Arg Met Ala Asp Asp
                   85                  90                  95
   Tyr Glu Lys Gln Leu Lys Thr Leu Asn Asp Glu Leu Val Glu Leu Gln
               100                 105                 110
   Gln His Thr Ser Glu Lys Ala Ser Ala Thr Ile Ala Lys Leu Lys Glu
           115                 120                 125
   Asp Ile Lys Lys Leu Asp Glu Glu Val Gly Thr Leu His Glu Lys Leu
       130                 135                 140
   Lys Gly Ile Lys Gln Asp Phe Glu Lys Val Lys Arg Asp Leu Cys Val
   145                 150                 155                 160
   Thr Tyr Leu Asn Ser Asn Gln Met Ser Lys Ala Lys Ala Lys Leu Lys
                   165                 170                 175
   Glu Met Ala Ser Thr Tyr Leu Ile Glu Ile Val Gln Gln Gln Leu Asn
               180                 185                 190
   Lys Ser Asn Ala Asn Ile Met Pro Met Leu Glu Phe Ser Ala Ala Ile
           195                 200                 205
   Pro Asp Leu Asp Asp Met Gly Glu Ala Tyr Lys Glu Ile Tyr Lys Phe
       210                 215                 220
   Leu Glu Glu Gln Lys Arg Leu Glu Gly Glu Asp Ser Val Leu Leu Glu
   225                 230                 235                 240
   Ala Thr Val Leu Lys Met Asn Ala Ser Leu Lys Glu Gly Ser Asn Ile
                   245                 250                 255
   Thr Asp Glu Arg Arg Thr Gln Ile Glu Gly Leu Leu Lys Asp Leu Ala
               260                 265                 270
   Thr Lys Ser Thr Ile Val Phe Ser Thr Trp Thr Lys Glu Leu Lys Lys
           275                 280                 285
   Ile Asn Asp Ala Val Val Ile Lys Asn Ala Leu Asp His Met Phe Val
       290                 295                 300
   Ser Gln Met Lys Val Phe Gly Ala Leu Val Gly Asp Thr Ser Asp Phe
   305                 310                 315                 320
   Gly Ser Ile Arg Asn Phe Val Lys Leu Thr Val Val Cys Asn Asn Tyr
                   325                 330                 335
   Tyr Lys Val Ala Ala Tyr Lys Glu Leu Ile Asp Arg Lys Ile Gly Asn
               340                 345                 350
   Ala Leu Gly Thr Ile Met Phe Asp Leu Leu Thr Leu Glu Val Asn Glu
           355                 360                 365
   Met Lys Phe Asp Pro His Val Pro Asp Glu Ile Pro Lys Leu Phe Glu
       370                 375                 380
   Ala Thr Leu Ser Ser Leu Pro Asn Ser Leu Thr Glu Leu Arg Thr Cys
   385                 390                 395                 400
   Leu Gly Lys Val Gln Ile Tyr Asn Lys Lys Thr Asn Lys Cys Val Val
                   405                 410                 415
   Ala Thr Gly Asn Asp Phe Asp Val His Lys Asp Lys Leu Gly Asp Phe
               420                 425                 430
   Tyr Arg Val Val Val Ala Asp Tyr Gly Cys Thr Ser Phe Arg Leu Glu
           435                 440                 445
   Ala Ser Gly Asp Lys Ala Ser Val Arg Ile Val Thr Pro Ser Gly Asn
       450                 455                 460
   Pro Met Ser Asn Val Asn Leu His Leu Glu Gly Asn Ser Leu His Asn
   465                 470                 475                 480
   Tyr Val Ala Thr Pro Lys Ser Asn Lys Pro Asp Arg Thr Pro Ser Ser
                   485                 490                 495
   Ser Asp Glu Trp Ile Leu Asp Ala Asn Tyr Asn Asn Asp Thr Ile Lys
               500                 505                 510
   Ile Glu Ser Gln Phe Ser Asp Tyr Lys Thr Lys Lys Thr Glu Val Asp
           515                 520                 525
   His Leu Leu Val Arg Asp Ile Asn His Leu Pro His Val Leu Val Ala
   530 535 540
   Arg Tyr Gly Phe Met Gly Leu Lys Asn Ser Asp Ala Lys Asp Thr Ile
   545 550 555 560
   Glu Trp Asn Leu Lys Cys Gly Ser
   565
<210> 14
   <211> 316
   <212> PRT
   <213> Aedes aegypti
<400> 14
   Met Glu Thr Ser Leu Pro Ile Thr Val Val Phe Leu Ile Val Leu Ile
   1 5 10 15
   Thr Gly Ala Gln Thr Lys Pro Thr Gln Gly Ser Cys Thr Leu Thr Asp
   20 25 30
   Glu Asp Ile Ser Asp Ile Lys Ser Ala Val Gln Lys Ala Ser Lys Ala
   35 40 45
   Ala Val Asn Asp Ile Val Leu Asp Pro Thr Leu Ile Asp Lys Cys Pro
   50 55 60
   Met Leu Glu Lys Ile Thr Ala Ser Leu Lys Ser Val Ala Thr Glu Ile
   65 70 75 80
   Val Gln Met Arg Asp Ser Ala Ile Ser Thr Asp Gln Val Asp Gln Leu
   85 90 95
   Lys Gln Asn Phe Glu Asp Gln Val Asn Gln Ile Val Lys Ser Arg Asp
   100 105 110
   Ile Phe Glu Lys Gln Ser Gly Thr Gln Ala Thr Lys Glu His Gly Glu
   115 120 125
   Met Leu Glu Arg Met Thr Ala Leu Gln Val Lys Val Thr Glu Leu Glu
   130 135 140
   Gln Gln Ile Ala Lys Gln Thr Ala Ser Met Tyr Glu Asp Met Ala Glu
   145 150 155 160
   Leu Ile Phe Gln Arg Leu Gln Met Asn Ser Thr Glu Ser Val Arg Ser
   165 170 175
   Tyr Thr Lys His Met Met Glu Glu Lys Leu Glu Glu Leu Met Asn Lys
   180 185 190
   Leu Glu Thr Asn Tyr Arg Ile Tyr Leu Gly Ala Leu Arg Phe Leu Asn
   195 200 205
   His Met Asn Asp Gln Glu Leu Ile Gly Lys Val Phe Asp Gly Ile Leu
   210 215 220
   Lys Arg Leu Gly Asp Met Lys Ala Asp Ser Asp Asp Val Lys Glu Asn
   225 230 235 240
   Gly Arg Asn Leu Leu Val Asn Leu Leu Cys Trp Thr Val Asn Asn Asp
   245 250 255
   Phe Leu Gly Lys Lys Tyr Lys Glu Arg Gln Val Asp Leu Tyr Arg Met
   260 265 270
   Ala Leu Lys Phe Tyr Pro Lys Thr Tyr Glu Lys Ala Ala Asn Glu Ala
   275 280 285
   Asp Val Arg Ser Arg Gln Phe Cys Glu Glu Asn Phe Pro Ala Asn Leu
   290 295 300
   Ile Thr Trp Phe Ala Val Ser Trp Asn Asp Arg Gly
   305 310 315
<210> 15
   <211> 215
   <212> PRT
   <213> Aedes aegypti
<400> 15
   Met Lys Tyr Leu Leu Thr Phe Leu Met Ala Leu Ser Leu Val Asn Leu
   1 5 10 15
   Met Leu Thr Arg Pro Thr Pro Glu Asp Asp Gly Gly Thr Ser Glu Glu
   20 25 30
   Pro Gln Thr Gln Glu Thr Thr Gly Ser Asp Glu Lys Asn Gly Ala Ser
   35 40 45
   Glu Glu Pro Asn Ala Asp Asp Ala Ser Lys Pro Asp Asp Val Glu Glu
   50 55 60
   Lys Gly Asp Asp Asp Thr Ala Lys Lys Glu Asp Asp Gly Glu Ser Lys
   65 70 75 80
   Asp Gly Glu Gly Ser Glu Lys Ser Asp Lys Glu Lys Gly Glu Pro Lys
   85 90 95
   Asn Asp Pro Arg Glu Thr Tyr Asn Lys Val Ile Glu Gln Leu Asp Gln
   100 105 110
   Ile Lys Val Asp Asn Val Glu Asp Gly His Glu Arg Ser Glu Leu Ala
   115 120 125
   Ala Asp Ile Gln Arg Tyr Leu Arg Asn Pro Ile Val Asp Val Ile Gly
   130 135 140
   Ser Ala Gly Asp Phe Ser Lys Ile Ala Lys Cys Phe Lys Ser Met Val
   145 150 155 160
   Gly Asp Ala Lys Lys Ala Ile Glu Glu Asp Val Lys Gly Phe Lys Glu
   165 170 175
   Cys Thr Ala Lys Lys Asp Ser Asn Ala Tyr Gln Cys Ser Gln Asp Arg
   180 185 190
   Ser Thr Val Gln Asp Lys Ile Ala Lys Met Ser Ser Lys Ile Ala Ser
   195 200 205
   Cys Val Ala Ser Asn Arg Ser
   210 215
<210> 16
   <211> 183
   <212> PRT
   <213> Aedes albopictus
<400> 16
   Met His Ser Leu Lys Ser Ser Pro Leu Leu Ala Ala Val Phe Leu Ala
   1               5                   10                  15
   Leu His Val Thr Gly Ala Pro Phe Trp Asn Ala Lys Asn Pro Asp Glu
               20                  25                  30
   Leu Gln Ser Ile Ala Ala Arg Cys Met Asp Glu Trp Ser Pro Lys Ala
           35                  40                  45
   Lys Asp Pro Lys Ala Ala Leu Lys Asn Trp Lys Glu Trp Arg Leu Gln
       50                  55                  60
   Pro Ser Asn Asp Glu Ala Thr Lys Cys Tyr Thr Lys Cys Met Leu Glu
   65                  70                  75                  80
   Asn Ile Gly Phe Tyr Glu Pro Ala Glu Lys Arg Leu Lys Gly Val Arg
                   85                  90                  95
   Ile Met Gln Gln Trp Glu Thr Phe Ser Arg Tyr Gln Ser Ala Asp Arg
               100                 105                 110
   Glu Lys Val His Asp Leu Thr Asp Thr Phe Asn Phe Ile Arg Pro Leu
           115                 120                 125
   Lys Ser Ser Ser Cys Thr Asp Val Phe Asn Ala Tyr Lys Asp Val His
       130                 135                 140
   Ala Arg His Leu Glu Thr Ile Lys Ala Ile Leu Phe Cys Asp Gly Lys
   145                 150                 155                 160
   Ser Ala Glu Lys Tyr Tyr Lys Asp Lys Gly Lys Thr Ser Lys Gln Lys
                   165                 170                 175
   Lys Val Leu Cys Thr Gly Ser
               180
<210> 17
   <211> 322
   <212> PRT
   <213> Aedes albopictus
<400> 17
   Met Lys Thr Ser Leu Pro Ile Val Val Leu Leu Thr Ala Val Ile Ser
   1               5                   10                  15
   Gly Val His Pro Asn Pro Thr Pro Lys Ser Cys Thr Val Ser Glu Glu
               20                  25                  30
   Asp Leu Thr Thr Ile Arg Asn Ala Ile Gln Lys Ala Ser Arg Ala Ser
           35                  40                  45
   Leu Asp Asp Val Asn Leu Asp Glu Asp Leu Ile Ala Lys Cys Pro Leu
       50                  55                  60
   Leu Lys Thr Ile Thr Ala Ser Leu Lys Ser Val Ala Ser Glu Ile Ala
   65                  70                  75                  80
   Thr Leu Lys Asp Thr Gly Ile Ser Glu Glu Gln Val Asp Glu Leu Lys
                   85                  90                  95
   Gln Ser Tyr Glu Gln Gln Val Asn Glu Ile Val Lys Ser Arg Asp Ile
               100                 105                 110
   Phe Glu Lys Gln Ser Gly Gly Asp Val Met Lys Glu Gln Gly Ala Met
           115                 120                 125
   Ile Asn Arg Met Thr Glu Leu Gln Val Gln Val Ala Gln Leu Gln Gln
       130                 135                 140
   Gln Ile Gly Glu Gln Thr Ser Arg Met Tyr Asp Asp Met Ala Glu Leu
   145                 150                 155                 160
   Ile Phe Gln Arg Leu Ala Met Asn Ser Thr Asp Ser Ile Arg Asn Tyr
                   165                 170                 175
   Thr Ala His Met Met Glu Gln Lys Leu His Thr Leu Met Thr Lys Leu
               180                 185                 190
   Glu Thr Asn Tyr Arg Ile Phe Leu Gly Ala Leu Arg Tyr Leu Asp His
           195                 200                 205
   Leu Gly Asp Gln Pro Leu Ile Asp Lys Val Phe Asp Gly Ile Leu Lys
       210                 215                 220
   Arg Leu Asp Glu Met Ser Leu Glu Thr Asn Lys Glu Arg Glu Asn Gly
   225                 230                 235                     240
   Lys Tyr Val Leu Val Asn Leu Leu Cys Trp Thr Val Asn Asn Arg Phe
                   245                 250                 255
   Leu Thr Glu Lys Tyr Arg Lys Lys Gln Leu Glu Leu Phe Arg Ile Ala
               260                 265                 270
   Leu Lys Phe Tyr Pro Lys Thr Gly Asn Lys Glu Ala Asn Glu Ala Asp
           275                 280                 285
   Ile Arg Gly Arg Gln Phe Cys Asp Ala Asn Phe Pro Val Asn Val Ile
       290                 295                 300
   Thr Trp Phe Ala Val Ser Arg Ala Ala Glu Gly Trp Gly Leu Arg Gly
   305                 310                 315                     320
   Thr Leu
<210> 18
   <211> 614
   <212> PRT
   <213> Aedes aegypti
<400> 18
   Met Ser Thr Leu Lys Lys Ile Ser Asp Glu Asp Arg Glu Ser Lys Phe
   1               5                   10                  15
   Gly Tyr Val Phe Ala Val Ser Gly Pro Val Val Thr Ala Glu Arg Met
               20                  25                  30
   Ser Gly Ser Ala Met Tyr Glu Leu Val Arg Val Gly Tyr Tyr Glu Leu
           35                  40                  45
   Val Gly Glu Ile Ile Arg Leu Glu Gly Asp Met Ala Thr Ile Gln Val
       50                  55                  60
   Tyr Glu Glu Thr Ser Gly Val Thr Val Gly Asp Pro Val Leu Arg Thr
   65                  70                  75                  80
   Gly Lys Pro Leu Ser Val Glu Leu Gly Pro Gly Ile Met Gly Ser Ile
                   85                  90                  95
   Phe Asp Gly Ile Gln Arg Pro Leu Lys Asp Ile Asn Glu Leu Thr Ser
   100 105 110
   Ser Ile Tyr Ile Pro Lys Gly Val Asn Ile Pro Cys Leu Ser Arg Thr
   115 120 125
   Gln Ser Trp Gly Phe Asn Pro Leu Asn Val Lys Val Gly Ser His Ile
   130 135 140
   Thr Gly Gly Asp Leu Tyr Gly Leu Val His Glu Asn Thr Leu Val Lys
   145 150 155 160
   His Lys Leu Leu Val Pro Pro Arg Ala Lys Gly Thr Val Arg Tyr Ile
   165 170 175
   Ala Pro Pro Gly Asn Tyr Thr Val Asp Asp Ile Ile Leu Glu Thr Glu
   180 185 190
   Phe Asp Gly Glu Ile Asn Lys Trp Ser Met Leu Gln Val Trp Pro Val
   195 200 205
   Arg Gln Pro Arg Pro Val Thr Glu Lys Leu Pro Ala Asn His Pro Leu
   210 215 220
   Leu Thr Gly Gln Arg Val Leu Asp Ser Leu Phe Pro Cys Val Gln Gly
   225 230 235 240
   Gly Thr Thr Ala Ile Pro Gly Ala Phe Gly Cys Gly Lys Thr Val Ile
   245 250 255
   Ser Gln Ala Leu Ser Lys Tyr Ser Asn Ser Asp Val Ile Ile Tyr Val
   260 265 270
   Gly Cys Gly Glu Arg Gly Asn Glu Met Ser Glu Val Leu Arg Asp Phe
   275 280 285
   Pro Glu Leu Ser Val Glu Ile Asp Gly Val Thr Glu Ser Ile Met Lys
   290 295 300
   Arg Thr Ala Leu Val Ala Asn Thr Ser Asn Met Pro Val Ala Ala Arg
   305 310 315 320
   Glu Ala Ser Ile Tyr Thr Gly Ile Thr Leu Ser Glu Tyr Phe Arg Asp
   325 330 335
   Met Gly Tyr Asn Val Ser Met Met Ala Asp Ser Thr Ser Arg Trp Ala
   340 345 350
   Glu Ala Leu Arg Glu Ile Ser Gly Arg Leu Ala Glu Met Pro Ala Asp
   355 360 365
   Ser Gly Tyr Pro Ala Tyr Leu Gly Ala Arg Leu Ala Ser Phe Tyr Glu
   370 375 380
   Arg Ala Gly Arg Val Lys Cys Leu Gly Asn Pro Glu Arg Glu Gly Ser
   385 390 395 400
   Val Ser Ile Val Gly Ala Val Ser Pro Pro Gly Gly Asp Phe Ser Asp
   405 410 415
   Pro Val Thr Ser Ala Thr Leu Gly Ile Val Gln Val Phe Trp Gly Leu
   420 425 430
   Asp Lys Lys Leu Ala Gln Arg Lys His Phe Pro Ser Ile Asn Trp Leu
   435 440 445
   Ile Ser Tyr Ser Lys Tyr Met Arg Ala Leu Asp Asp Phe Tyr Asp Lys
   450 455 460
   Asn Phe Gln Glu Phe Val Pro Leu Arg Thr Lys Val Lys Glu Ile Leu
   465 470 475 480
   Gln Glu Glu Glu Asp Leu Ser Glu Ile Val Gln Leu Val Gly Lys Ala
   485 490 495
   Ser Leu Ala Glu Thr Asp Lys Ile Thr Leu Glu Val Ala Lys Leu Leu
   500 505 510
   Lys Asp Asp Phe Leu Gln Gln Asn Ser Tyr Ser Ala Tyr Asp Arg Phe
   515 520 525
   Cys Pro Phe Tyr Lys Thr Val Gly Met Leu Arg Asn Met Ile Gly Phe
   530 535 540
   Tyr Asp Met Ala Arg His Ala Val Glu Thr Thr Ala Gln Ser Glu Asn
   545 550 555 560
   Lys Ile Thr Trp Asn Val Ile Arg Asp Ser Met Gly Asn Ile Leu Tyr
   565 570 575
   Gln Leu Ser Ser Met Lys Phe Lys Asp Pro Val Lys Asp Gly Glu Ala
   580 585 590
   Lys Ile Lys Ala Asp Phe Asp Gln Leu Tyr Glu Asp Leu Gln Gln Ala
   595 600 605
   Phe Arg Asn Leu Glu Asp
   610
<210> 19
   <211> 564
   <212> PRT
   <213> Aedes albopictus
<400> 19
   Met Ala Gly Lys Pro Gly Ile Gln Leu Phe Val Ile Phe Ile Leu Leu
   1               5                   10                  15
   Ser Ser Phe Ala Ala Val Val Trp Thr Thr Asp Asn Met Pro Ala Asp
               20                  25                  30
   Lys Asp Val Ser Lys Leu Phe Pro Leu Thr Leu Ile His Ile Asn Asp
           35                  40                  45
   Leu His Ala Arg Phe Asp Glu Thr Asn Met Lys Ser Asn Ala Cys Thr
       50                  55                  60
   Ala Lys Asp Gln Cys Ile Ala Gly Ile Ala Arg Val Tyr Gln Lys Ile
   65                  70                  75                  80
   Gln Asp Leu Leu Lys Glu Tyr Lys Ser Lys Asn Ala Ile Tyr Leu Asn
                   85                  90                  95
   Ala Gly Asp Asn Phe Gln Gly Thr Leu Trp Tyr Asn Leu Leu Arg Trp
               100                 105                 110
   Gln Val Thr Ala Asp Phe Ile Thr Lys Leu Lys Pro Thr Ala Met Thr
           115                 120                 125
   Leu Gly Asn His Glu Phe Asp His Thr Pro Lys Gly Leu Ala Pro Tyr
       130                 135                 140
   Leu Ala Glu Leu Asp Lys Ala Gly Ile Pro Thr Leu Val Ala Asn Leu
   145                 150                 155                 160
   Val Met Asn Asp Asp Pro Asp Leu Lys Ser Ser Lys Ile Gln Lys Ser
                   165                 170                 175
   Ile Lys Val Thr Val Gly Gly Lys Thr Ile Gly Ile Ile Gly Val Leu
               180                 185                 190
   Tyr Asp Lys Thr His Glu Ile Ala Gln Thr Gly Lys Val Thr Leu Ser
           195                 200                 205
   Asn Ala Val Glu Thr Val Lys Arg Glu Ala Ala Ala Leu Lys Lys Asp
       210                 215                 220
   Lys Val Asp Ile Ile Val Val Leu Ser His Cys Ser Tyr Asp Glu Asp
   225                 230                 235                 240
   Lys Lys Ile Ala Lys Glu Ala Gly Gln Asp Ile Asp Val Ile Val Gly
                   245                 250                 255
   Ala His Ser His Ser Phe Leu Tyr Ser Lys Glu Ser Asn Lys Pro Tyr
               260                 265                 270
   Asp Gln Lys Asp Lys Ile Glu Gly Pro Tyr Pro Thr Ile Val Glu Ser
           275                 280                 285
   Asn Asn Lys Arg Lys Ile Pro Ile Val Gln Ala Lys Ser Phe Gly Lys
       290                 295                 300
   Tyr Val Gly Arg Leu Thr Leu Tyr Phe Asp Asn Glu Gly Glu Val Lys
   305                 310                 315                 320
   His Trp Glu Gly Tyr Pro Glu Phe Ile Asp Asn Lys Val Lys Gln Asp
                   325                 330                 335
   Pro Lys Ile Leu Glu Ala Leu Ile Pro Trp Arg Lys Lys Val Gln Glu
               340                 345                 350
   Ile Gly Ser Thr Lys Val Gly Glu Thr Thr Ile Glu Leu Asp Arg Asp
           355                 360                 365
   Ser Cys Arg Asp Lys Glu Cys Thr Leu Gly Val Leu Tyr Ala Asp Ala
       370                 375                 380
   Phe Ala Asp His Tyr Thr Asn Ser Ser Phe Arg Pro Phe Ala Ile Ile
   385                 390                 395                 400
   Gln Ala Gly Asn Phe Arg Asn Pro Ile Lys Val Gly Lys Ile Thr Asn
                   405                 410                 415
   Gly Asp Ile Ile Glu Ala Ala Pro Phe Gly Ser Thr Ala Asp Leu Ile
               420                 425                 430
   Arg Leu Lys Gly Asp Ser Leu Trp Ala Val Ala Glu His Ser Phe Ala
           435                 440                 445
   Leu Asp Asp Glu Asn Arg Thr Asn Cys Leu Gln Val Ser Gly Leu Arg
       450                 455                 460
   Ile Val Ile Asp Pro Ser Lys Lys Ile Gly Ser Arg Val Val Lys Ile
   465                 470                 475                 480
   Asp Val Met Asp Asn Arg Asn Pro Lys Ser Glu Asp Leu Lys Pro Leu
                   485                 490                 495
   Asp Lys Asn Ala Glu Tyr Phe Ile Ala Leu Pro Ser Tyr Leu Ala Asp
               500                 505                 510
   Gly Lys Asp Gly Phe Ser Ala Met Lys Lys Ala Thr Ala Arg Trp Thr
           515                 520                 525
   Gly Pro Leu Asp Ser Asp Val Phe Lys Ser Tyr Val Glu Lys Ile Lys
       530                 535                 540
   Lys Val Asp Lys Leu Lys Trp Gly Arg Val Ile Val Cys Lys Ala Gly
   545                 550                 555                 560
   Ser Pro Cys Thr
<210> 20
   <211> 567
   <212> PRT
   <213> Aedes albopictus
<400> 20
   Met Lys Trp Ser Val Tyr Ile Ala Leu Leu Val Phe Ala Phe Leu Thr
   1               5                   10                  15
   Ser Pro Val Phe Ser Glu Glu Asn Cys Asn Ile Pro Glu Ser Glu Leu
               20                  25                  30
   Ser Lys Ile Asp Asp Val Leu Arg His Met Glu Lys Pro Ile Tyr Ser
           35                  40                  45
   Glu Asp His Tyr Thr Ser Asn Asn Glu Glu Cys Thr Asn Leu Leu Asn
       50                  55                  60
   Gly Ile His Ala Gln Leu Arg Arg Leu Thr Gln Arg Tyr Lys Leu Met
   65                  70                  75                  80
   Asn Lys Gly Tyr Val Lys Val Glu Glu Tyr Lys Arg Met Ala Glu Asp
                   85                  90                  95
   Tyr Glu Asn Gln Leu Lys Thr Leu Asn Ala Glu Leu Leu Glu Leu Gln
               100                 105                 110
   Glu His Thr Ser Asp Lys Ala Asn Ala Ala Ile Ala Lys Leu Lys Glu
           115                 120                 125
   Asp Ile Lys Lys Leu Asp Glu Asp Val Asp Thr Leu His Asn Lys Leu
       130                 135                 140
   Lys Gly Ile Lys Gln Asp Phe Glu Lys Val Lys Arg Asp Leu Cys Leu
   145                 150                 155                 160
   Thr Tyr Leu Asn Ser Asn Gln Met Ser Asn Ala Lys Ala Lys Val Lys
                   165                 170                 175
   Glu Met Ala Ser Thr Tyr Leu Ile Glu Ile Ile Gln Gln Arg Leu Asn
               180                 185                 190
   Thr Lys Tyr Ala Asn Ile Ile Pro Met Leu Asp Phe Ser Thr Ala Ile
           195                 200                 205
   Pro Asp Leu Asp Asp Arg Gly Glu Ala Tyr Lys Glu Ile Tyr Lys Phe
   210 215 220
   Ile Glu Thr His Glu Arg Leu Asp Gly Glu Asp Ala Val Leu Leu Glu
   225 230 235 240
   Ala Ser Leu Leu Lys Met Asn Ala Thr Leu Lys Glu Gly Ser Asn Ile
   245 250 255
   Thr Asp Glu Arg Arg Thr Glu Ile Glu Lys Met Leu Lys Glu Leu Ala
   260 265 270
   Glu Lys Ser Ala Val Val Phe Lys Thr Trp Ser Thr Glu Leu Lys Gly
   275 280 285
   Ile Glu Asp Thr Ile Ile Lys Tyr Ala Leu Asp His Leu Phe Val Asn
   290 295 300
   Gln Met Lys Val Phe Gly Gly Ile Val Gly Asp Thr Phe Glu Phe Ala
   305 310 315 320
   Pro Ile Arg His Leu Leu Lys Leu Leu Val Val Cys Asn Asn Tyr Tyr
   325 330 335
   Lys Val Ala Ala Tyr Lys Glu Leu Ile Asp Arg Lys Ile Gly Asn Val
   340 345 350
   Leu Gly Thr Ile Met Phe Asp Leu Thr Thr Leu Glu Ala Asn Glu Met
   355 360 365
   Ser Phe Asp Leu His Val Pro Asp Glu Ile Pro Lys Leu Phe Asn Ala
   370 375 380
   Thr Leu Gly Ser Leu Pro Asn Ser Leu Thr Gln Leu Leu Pro Cys Leu
   385 390 395 400
   Asn Lys Val His Val Tyr Asn Ala Lys Thr Asn Met Cys Ile Val Ala
   405 410 415
   Pro Glu Asp Arg Phe Asp Val Gln Gln Glu Lys Leu Thr Asp Phe His
   420 425 430
   Arg Val Val Leu Ala Lys Tyr Gly Cys Thr Ala Phe Arg Leu Glu Ser
   435 440 445
   Ser Pro Asn Lys Ala Ser Val Lys Phe Val Lys Pro Ser Gly Asn Ala
   450 455 460
   Leu Ser Ser Ile Asn Leu Gln Leu Glu Asn Asp Gln Trp His Ser His
   465 470 475 480
   Val Gly Thr Pro Thr Ala Asn Lys Pro Asp Arg Lys Pro Ser Ser Ser
   485 490 495
   Asp Glu Trp Ile Leu Asp Ala Asn Tyr Val Asn Asp Thr Val Lys Ile
   500 505 510
   Gln Ser Glu Phe Asn Glu Tyr Lys Ala Ser Gln Ala Glu Val Asp His
   515 520 525
   Leu Leu Val Met Asp Val Lys Tyr Leu Pro His Val Val Val Gly Arg
   530 535 540
   Tyr Gly Val Arg Gly Leu Lys Arg Ser Ser Ala Lys Asp Thr Ile Glu
   545 550 555 560
   Trp Tyr Leu Lys Cys Ala Ser
   565
<210> 21
   <211> 256
   <212> PRT
   <213> Aedes albopictus
<400> 21
   Met Ala Phe Asn Gly Ile Ala Leu Leu Ile Thr Ala Thr Ile Phe Ile
   1 5 10 15
   Gly Ser Cys Tyr Ala Asn Tyr Cys Asp Ser Ser Leu Cys Arg Gln Gly
   20 25 30
   Pro His Val Ala Cys Asn Ala Pro Gln Gln Phe Gly Pro Ala Cys Gly
   35 40 45
   Asn Asn Arg Lys Phe Val Pro Met Asp Ser Lys Leu Lys Thr Ile Ile
   50 55 60
   Leu Asn Thr His Asn Lys Leu Arg Ala Glu Ile Ala Asn Gly Met His
   65 70 75 80
   Gly Phe Pro Gln Ala Ala Arg Met Pro Thr Leu Val Trp Asp Asp Glu
   85 90 95
   Leu Ala His Ile Ala Ser Phe Asn Ala Arg Lys Cys Ile Phe Ala His
   100 105 110
   Asp Lys Cys Arg Asn Thr Arg Gln Phe Lys Phe Ser Gly Gln Asn Leu
   115 120 125
   Ala Ile Thr Thr Phe Tyr Gly Phe Asn Phe Gln Ala Gly Asp Arg Ala
   130 135 140
   Glu Asn Phe Thr Gln Glu Trp Phe Asn Glu His Lys Asp Cys Pro Lys
   145                 150                 155                 160
   Ser Tyr Val Asp Ala Tyr Pro Ser Ser His Arg Gly Pro Gln Ile Gly
                   165                 170                 175
   His Phe Thr Gln Leu Val Asn Asp Arg Thr Trp Lys Val Gly Cys Ser
               180                 185                 190
   Met Met His Tyr Ile Thr Asn Gly Lys Met Ile Asn Tyr Tyr Leu Val
           195                 200                 205
   Cys Asn Tyr Thr Met Thr Asn Met Ile Gly Glu Pro Ile Tyr Thr Lys
       210                 215                 220
   Gly Arg Thr Gly Ser Lys Cys Glu Thr Gly Gln Asn Pro Gln Phe Lys
   225                 230                 235                 240
   Gly Leu Cys Ser Pro Arg Glu Lys Val Lys Ser Glu Ser Tyr Asn Gly
                   245                 250                 255
<210> 22
   <211> 422
   <212> PRT
   <213> Aedes albopictus
<400> 22
   Met Cys Ser Thr Gly Leu Cys Leu Val Phe Phe Ile Ala Gln Ala Val
   1               5                   10                  15
   Phe Leu Met Asn Tyr Ser Glu Gln Gln Thr Thr Val Val Met Glu Asn
               20                  25                  30
   Gly Ala Ile Ser Glu Lys Glu Thr Asn Val Asp Glu Val Met Thr Gln
           35                  40                  45
   Phe Ile Met Lys Phe Tyr Thr Lys Arg Phe Val Glu Gly Gln Asn Leu
       50                  55                  60
   Val Val Ala Pro Leu Leu Ile Phe Arg Val Phe Met Ser Met Tyr Gly
   65                  70                  75                  80
   Glu Met Asp Ala Ser Ala Lys Phe Asp Leu His Ser Leu Val Gly Ile
                   85                  90                  95
   Pro Gln Glu Ala Ser Ala Glu Lys Met Ser Glu Phe Glu Ala Phe Ala
               100                 105                 110
   Asn Lys Tyr Ala Leu Pro Val Gly Val Gln Arg Asn Leu Val Glu Thr
           115                 120                 125
   Arg Leu Tyr Tyr Asp Lys Ser Ile Gly Lys Ile Arg Ser Ser Leu Glu
   130 135 140
   Ala Lys Ser Leu Lys Pro Phe Pro Thr Asn Phe Ala Asp Lys Gln Thr
   145 150 155 160
   Phe Cys Asn Glu Val Asn Thr Trp Ile Arg Asn Thr Pro Ile Asn Gly
   165 170 175
   Thr Asp Asp Leu Val His Asp Tyr Tyr Leu Asn Asn Glu Thr Ala Ala
   180 185 190
   Phe Val Ala Gly Ala Leu Ser Ile Asp Trp Asn Met Gln Leu Lys Thr
   195 200 205
   Ser Ser Asp Val Lys Ala Phe Glu Gly Glu Asn Val Lys Phe Leu Glu
   210 215 220
   Gly Ser Ile Ser Thr Arg Tyr Ala Lys Leu Asp Asn Leu Lys Val Glu
   225 230 235 240
   Val Val Glu Met Val Thr Asp Asn Leu Ser Gly Val Lys Leu Trp Leu
   245 250 255
   Ile Met Pro Asp Glu Ala Ser Ser Ile Lys Lys Phe Asn Asp Gln Leu
   260 265 270
   Ser Ile Ala Ser Ile Arg Gln Ile Glu Lys Gly Leu Thr Ala Leu Gln
   275 280 285
   Lys Glu Asp Val Ala Leu Thr Val Pro Met Val Thr Ile Glu Tyr Asn
   290 295 300
   Ser Gln Glu Asp Ala Tyr Val Thr Glu Val Phe Glu Val Phe Ser Ser
   305 310 315 320
   Leu Phe Ser Lys Pro Ala Val Lys Pro Trp Phe Arg Val Ser Val Lys
   325 330 335
   Asp Asp Leu Tyr Ala Val Lys Asn Phe Leu Met Lys Cys Ile Leu Arg
   340 345 350
   Phe Val Gly Ser Asp Ala Pro Ala Asp Ser Lys Gly Gln Ser Thr Glu
   355 360 365
   Lys Ala Val Ser Phe Asn Arg Pro Phe Val Met Met Ile Leu Ser Lys
   370 375 380
   Glu Ser Asn Val Pro Ile Leu Leu Ala Asn Tyr Phe Ser Pro Lys Asp
   385 390 395 400
   Lys Leu Arg Ala Leu Glu Ala Lys Glu Arg His Leu Arg Met Lys Ala
   405 410 415
   Lys Glu His Leu Asp Leu
   420
<210> 23
   <211> 533
   <212> PRT
   <213> Aedes albopictus
<400> 23
   Met Lys Ile Leu Leu Ala Val Val Phe Val Leu Asn Leu Thr Asn Leu
   1               5                   10                  15
   Ala Val Pro Gln His Leu Ile Thr Ser Ser Pro Ser Leu Pro Glu Ser
               20                  25                  30
   Lys Pro Val Gly Arg Arg Pro Thr Tyr Glu Glu Tyr Lys Gln Gln Arg
           35                  40                  45
   Glu Ser Phe Leu Gln Thr Glu Asp His His Leu Leu Gly Ala Asn Val
       50                  55                  60
   Thr Leu Thr Glu Asn Glu Gln Leu Val Asn Lys Phe Ile Met Gln Met
   65                  70                  75                  80
   Lys Leu Asp Glu Met Glu Lys Gly Phe Asn Asp Ser Tyr Asn Phe Ile
                   85                  90                  95
   Pro Ala Arg His Ile Phe Glu Val Leu Asp Arg Phe Gly Gln Ser Lys
               100                 105                 110
   Val Phe Asn Val Ile Arg Arg Leu Pro Lys Gly Gly Val Leu His Ala
           115                 120                 125
   His Asp Met Ala Leu Gly Ser Thr Asp Leu Ile Val Asn Ala Thr Tyr
       130                 135                 140
   Leu Glu Asn Leu Trp Gln Lys Gly Asn Phe Gly Leu Asn His Gly Pro
   145                 150                 155                 160
   Glu Phe Lys Phe Ser Arg Glu Arg Pro Gly Lys Glu Trp Ser Leu Val
                   165                 170                 175
   Ser Glu Ile Arg Gln Trp Met Thr Asn Glu Val Tyr Asp Ala Lys Val
               180                 185                 190
   Ala Glu Val Phe Ser Leu Tyr Asn Ala Asp Pro Leu Asn Ala Tyr Lys
           195                 200                 205
   Ser Leu Asp Asn Val Trp Ser Lys Phe Gln Asn Leu Phe Ala Cys Leu
       210                 215                 220
   Ala Pro Leu Ile Thr Phe Ala Pro Val Trp Arg Gln Tyr Tyr His Asp
   225 230 235 240
   Ser Leu Lys Gln Phe Tyr Asp Asp His Val Gln Tyr Leu Glu Phe Arg
   245 250 255
   Gly Val Leu Pro Glu Val Tyr Asp Leu Asp Gly Lys Val Tyr Ser Ala
   260 265 270
   Glu Glu Ile Val Gln Leu Tyr Tyr Glu Glu Thr Glu Gln Phe Lys Ala
   275 280 285
   Lys Tyr Pro Asp Phe Ile Gly Val Lys Phe Ile Tyr Ala Pro Gly Arg
   290 295 300
   Tyr Ala Ser Asp Glu Glu Phe Gln Lys Leu Leu Asp Thr Thr Asn Arg
   305 310 315 320
   Leu His Lys Lys Phe Pro Asn Phe Leu Ala Gly Phe Asp Leu Val Gly
   325 330 335
   Gln Glu Asp Pro Gly Arg Ser Leu Phe Glu Phe Ala Pro Ala Leu Leu
   340 345 350
   Lys Leu Pro Ala Ser Ile Asn Phe Phe Phe His Ala Gly Glu Thr Asn
   355 360 365
   Trp Tyr Gly Met Lys Thr Asp Gln Asn Leu Val Asp Ala Val Leu Leu
   370 375 380
   Gly Thr Lys Arg Ile Gly His Gly Phe Ala Val Leu Lys His Pro Lys
   385 390 395 400
   Val Leu Lys Glu Ile Lys Arg Arg Gln Ile Cys Ile Glu Ile Asn Pro
   405 410 415
   Ile Ser Asn Gln Val Leu Lys Leu Val Gln Asp Gln Arg Asn His Pro
   420 425 430
   Ala Ala Leu Leu Phe Ser Asp Asn Tyr Pro Val Val Val Ser Ser Asp
   435 440 445
   Asp Pro Ser Phe Gly Arg Ser Thr Pro Leu Ser His Asp Phe Tyr Val
   450 455 460
   Ala Phe Thr Gly Ile Ala Ser Ala Lys Gln Asp Trp Arg Trp Leu Lys
   465 470 475 480
   Gln Leu Ala Leu Asn Ser Ile Glu Tyr Ser Ala Met Asn Ser Glu Glu
   485 490 495
   Lys Thr Val Ala Lys Glu Lys Trp Asn Gln Ala Trp Asp His Gln Phe
   500 505 510
   Ser Arg Leu Ala Val Asp Phe Val Ala Gly Lys Ile Leu Glu Asn Trp
   515 520 525
   Ile Met Lys Ile Val
   530
<210> 24
   <211> 389
   <212> PRT
   <213> Aedes aegypti
<400> 24
   Met Gln Pro Arg Ile Leu His Leu Thr Val Leu Ala Thr Ile Ile Gly
   1 5 10 15
   Val Ala Leu Thr Ala Asn Val Pro Ser Thr Pro Gly Arg Lys Leu Asn
   20 25 30
   Ile Pro Ala Phe Ser Asn Ala Gly Lys Thr Lys Gly Ile Glu Ile Trp
   35 40 45
   Arg Ile Glu Asn Phe Gln Pro Val Ala Val Pro Lys Ala Glu Tyr Gly
   50 55 60
   Lys Phe Tyr Thr Gly Asp Ser Tyr Leu Val Leu Asn Thr Asn Glu Asp
   65 70 75 80
   Lys Asn Lys Lys Lys Ser Tyr Asp Val His Phe Trp Leu Gly Leu Lys
   85 90 95
   Thr Thr Gln Asp Glu Ala Gly Ser Ala Ala Ile Leu Thr Val Gln Leu
   100 105 110
   Asp Asp Leu Leu Gly Gly Gly Pro Val Gln His Arg Glu Val Glu Gly
   115 120 125
   Ser Glu Ser Asp Leu Phe Leu Ser Tyr Phe Lys Gly Gly Ile Arg Tyr
   130 135 140
   Leu Glu Gly Gly Val Ala Ser Gly Phe Lys His Val Gln Thr Asn Ala
   145 150 155 160
   Ala His Pro Lys Arg Leu Phe His Val Lys Gly Ala Lys Asn Ile Arg
   165 170 175
   Leu Arg Gln Val Glu Leu Ala Val Ser Ala Met Asn Lys Gly Asp Cys
   180 185 190
   Phe Ile Leu Asp Ser Asp Arg Asp Val Phe Val Trp Val Gly Pro Lys
   195 200 205
   Ala Asn Arg Val Glu Lys Leu Lys Ala Ile Asn Val Ala Asn Asp Ile
   210 215 220
   Arg Asp Arg Asp His Asn Gly Arg Ala Thr Val His Ile Val Asp Glu
   225 230 235 240
   Phe Ser Thr Leu Ser Asp Gln Glu Ser Phe Phe Lys Ser Leu Gly Ser
   245 250 255
   Gly Ser Pro Ser Thr Val Pro Asp Gln Ser Thr Ala Lys Glu Asp Ala
   260 265 270
   Ala Phe Glu Lys Ala Asp Ala Ala Arg Val Glu Leu Tyr Lys Val Thr
   275 280 285
   Asp Ser Lys Ala Gly Lys Leu Ala Val Glu Pro Ile Thr Gln Lys Pro
   290 295 300
   Leu Lys Gln Glu Met Leu Lys Pro Asp Asp Ala Phe Ile Leu Asp Thr
   305 310 315 320
   Gly Ser Gly Leu Tyr Val Trp Ile Gly Lys Ser Ala Thr Gln Gln Glu
   325 330 335
   Lys Thr Gln Ser Leu Val Lys Ala Gln Glu Phe Ile Lys Asn Lys Lys
   340 345 350
   Tyr Pro Ala Trp Thr Pro Val Glu Arg Ile Val Gln Asn Ala Glu Thr
   355 360 365
   Ala Pro Phe Lys His Phe Phe Gln Thr Trp Arg Asp Ala Gly Ser Thr
   370 375 380
   Gly Ser Arg Leu Val
   385
<210> 25
   <211> 259
   <212> PRT
   <213> Artificial sequence
<220>
   <223> Protein recombinante
<400> 25
   Met Lys Ser Ile Val Ser Ile Thr Ile Thr Val Leu Ala Ile Ile Cys
   1               5                   10                  15
   Glu Gly Gln Ala Thr Asn Tyr Cys Asp Pro Ser Leu Cys Ala Arg Gly
               20                  25                  30
   Thr Pro His Ile Ala Cys Asn Gly Leu Ser Thr Leu Ser Arg Thr Cys
           35                  40                  45
   Gly Ala Gly Ser Phe Glu Val Ala Leu Asn Arg Ala Asp Gln Gln Leu
       50                  55                  60
   Ile Val Asp Leu His Asn Lys Leu Arg Ser Lys Val Ala Met Gly Gln
   65                  70                  75                  80
   Gln Lys Asn Ser Ala Gly Gln Arg Phe Gln Gln Ala Cys Arg Met Ala
                   85                  90                  95
   Thr Leu Gln Trp Asp Pro Glu Leu Ala His Ile Ala Ala Thr Asn Ala
               100                 105                 110
   Arg Arg Cys Val Tyr Gly His Asp Thr Cys Arg Asn Thr Ala Ser Met
           115                 120                 125
   Lys Phe Ala Gly Gln Asn Ile Ala Ile Lys Tyr Tyr Tyr Gly Met Thr
       130                 135                 140
   Phe Thr Asn Glu Gln Leu Leu Thr Gly Phe Ile Asn Ser Trp Phe Ser
   145                 150                 155                 160
   Glu Phe Lys Asp Ala Thr Pro Gln Gln Ile Ala Arg Tyr Pro Ala Asn
                   165                 170                 175
   Tyr Arg Gly Pro Ala Ile Gly His Phe Thr Gln Ile Val Ser Asp Arg
               180                 185                 190
   Thr Ser Arg Ile Gly Cys Ser Met Val Ser Tyr Asn Lys Asn Gly Phe
           195                 200                 205
   Ile Asn Lys Leu Phe Val Cys Asn Tyr Gly Leu Thr Asn Ile Ile Asn
       210                 215                 220
   Gln Pro Val Tyr Val Ala Gly Asn Val Cys Ser Gly Cys Thr Thr Gly
   225                 230                 235                 240
   Cys Asn Lys Val Phe Pro Gly Leu Cys Asn Thr Ala Glu Arg Val Ser
                   245                 250                 255
   Asn Asn Pro
<210> 26
   <211> 566
   <212> PRT
   <213> Artificial sequence
<220>
   <223> Protein recombinante
<400> 26
   Met Lys Val Tyr Ile Cys Gln Val Ile Phe Ser Phe Leu Ala Val Ser
   1               5                   10                  15
   Val Phe Cys Glu Glu Asn Cys Asn Ile Pro Glu Ser Glu Leu Ser Lys
               20                  25                  30
   Ile Asp His Val Leu Arg His Met Glu Lys Pro Ile Tyr Ser Glu Glu
           35                  40                  45
   Gln Phe Ala Ser Asp Asn Glu Glu Cys Thr Asn Leu Leu Asn Gly Ile
       50                  55                  60
   His Ala Gln Leu Arg Arg Leu Thr Gln Arg Tyr Lys Leu Met Asn Lys
   65                  70                  75                  80
   Gly Tyr Val Lys Val Glu Glu Tyr Gln Arg Met Ala Asp Asn Tyr Glu
                   85                  90                  95
   Lys Gln Leu Lys Thr Leu Asn Asp Glu Leu Val Glu Leu Gln Gln His
               100                 105                 110
   Thr Ser Glu Lys Ala Ser Ala Thr Ile Ala Lys Leu Lys Glu Asp Ile
           115                 120                 125
   Lys Lys Leu Asp Glu Glu Val Gly Thr Leu His Glu Lys Leu Lys Gly
       130                 135                 140
   Ile Lys Gln Asp Phe Glu Lys Val Lys Arg Asp Leu Cys Val Thr Tyr
   145                 150                 155                 160
   Leu Asn Ser Asn Gln Met Ser Lys Ala Lys Ala Lys Leu Lys Glu Met
                   165                 170                 175
   Ala Ser Thr Tyr Leu Ile Glu Ile Val Gln Gln Gln Leu Asn Lys Ser
               180                 185                 190
   Asn Ala Asn Ile Met Pro Met Leu Glu Phe Ser Ala Ala Ile Pro Asp
           195                 200                 205
   Leu Asp Asp Met Gly Glu Ala Tyr Lys Glu Ile Tyr Lys Phe Leu Glu
       210                 215                 220
   Glu Gln Lys Arg Leu Glu Gly Glu Asp Ser Val Leu Leu Glu Ala Thr
   225                 230                 235                 240
   Val Leu Lys Met Asn Ala Ser Leu Lys Glu Gly Ser Asn Ile Thr Asp
                   245                 250                 255
   Glu Arg Arg Thr Gln Ile Glu Gly Leu Leu Lys Asp Leu Ala Thr Lys
               260                 265                 270
   Ser Thr Ile Val Phe Ser Thr Trp Thr Lys Glu Leu Lys Lys Ile Asn
           275                 280                 285
   Asp Ala Val Val Ile Lys Asn Ala Leu Asp His Met Phe Val Ser Gln
       290                 295                 300
   Met Lys Val Phe Gly Ala Leu Val Gly Asp Thr Ser Asp Phe Gly Ser
   305                 310                 315                 320
   Ile Arg Asn Phe Val Lys Leu Thr Ile Val Cys Asn Asn Tyr Tyr Lys
                   325                 330                 335
   Val Ala Ala Tyr Lys Glu Leu Ile Asp Arg Lys Ile Gly Asn Ala Leu
               340                 345                 350
   Gly Thr Ile Met Phe Asp Leu Leu Thr Leu Glu Val Asn Glu Met Lys
           355                 360                 365
   Phe Asp Pro His Val Pro Asp Glu Ile Pro Lys Leu Phe Glu Ala Thr
       370                 375                 380
   Leu Ser Ser Leu Pro Asn Ser Leu Thr Glu Leu Arg Thr Cys Leu Gly
   385                 390                 395                 400
   Lys Val Gln Ile Tyr Asn Lys Lys Thr Asn Lys Cys Val Val Ala Thr
                   405                 410                 415
   Gly Asn Asp Phe Asp Val His Lys Asp Lys Leu Gly Asp Phe Tyr Arg
               420                 425                 430
   Val Val Val Ala Asp Tyr Gly Cys Thr Ser Phe Arg Leu Glu Ala Ser
           435                 440                 445
   Gly Asp Lys Ala Ser Val Arg Ile Val Thr Pro Ser Gly Asn Pro Met
       450                 455                 460
   Ser Asn Val Asn Leu His Leu Glu Gly Asn Ser Leu His Asn Tyr Val
   465                 470                 475                 480
   Ala Thr Pro Lys Ser Asn Lys Pro Asp Arg Thr Pro Ser Ser Ser Asp
                   485                 490                 495
   Glu Trp Ile Leu Asp Ala Asn Tyr Asn Asn Asp Thr Ile Lys Ile Glu
               500                 505                 510
   Ser Gln Phe Ser Asp Tyr Lys Thr Lys Lys Thr Glu Val Asp His Leu
           515                 520                 525
   Leu Val Arg Asp Ile Asn His Leu Pro His Val Leu Val Ala Arg Tyr
       530                 535                 540
   Gly Phe Met Gly Leu Lys Asn Ser Asp Ala Lys Asp Thr Ile Glu Trp
   545                 550                 555                 560
   Asn Leu Lys Cys Gly Ser
                   565